# EUROPEAN PATENT SPECIFICATION

(11) **EP 0 436 902 B1**
(45) Date of publication and mention of the grant of the patent: **28.06.1995**
(21) Application number: 90124970.6
(22) Date of filing: 29.10.1985
(51) Int. Cl.: C07D 401/12, C07D 401/14, A61K 31/505

(54) **5-Fluorouracil derivatives**
5-Fluoruracilderivate
Dérivés de 5-fluorouracile

(30) Priority: 30.10.1984 JP 229938/84; 30.11.1984 JP 254587/84; 17.01.1985 JP 7190/85; 25.03.1985 JP 59788/85; 09.05.1985 JP 98295/85; 30.08.1985 JP 192582/85; 03.09.1985 JP 195223/85
(43) Date of publication of application: 17.07.1991
(62) Divisional of application: 85113724.0
(73) Proprietor: OTSUKA PHARMACEUTICAL CO., LTD., Chiyoda-ku Tokyo-to (JP)
(72) Inventor: Fujii, Setsuro, Aig de Blanche 703, Nakagyo-ku, Kyoto-shi, Kyoto-fu (JP)
(74) Representative: VOSSIUS & PARTNER

(56) References cited:
- EP-A- 0 009 882
- EP-A- 0 129 984
- FR-A- 2 428 052
- GB-A- 2 066 812
- GB-A- 2 072 164
- GB-A- 2 117 766
- CHEMICAL ABSTRACTS, vol. 101, 1984, page 657, abstract no. 55487y, Columbus, Ohio, US; & JP-A-59 29 699 (FUNAI PHARM. IND.) 16-02-1984
- CHEMICAL ABSTRACTS, vol. 95, no. 15, 12th October 1981, page 654, abstract no. 132682v, Columbus, Ohio, US; & JP-A-81 46 867 (ONO PHARMACEUTICAL CO., LTD) 28-04-1981

## Description

This invention relates to novel 5-fluorouracil derivatives, process for preparing the same and pharmaceutical compositions containing the same.

FR-A-2 428 052 discloses 5-fluoro-(β-uridine or 2'-deoxy-β-uridine) derivatives which can be used as carcinostatic agents. Chem. Abstr. 101:55487y describes 2'-deoxy-5-fluorouridine ether derivatives exhibiting anticarcinogenic activity. GB-A-2 066 812 discloses 5'-acyl uridine derivatives possessing strong anti-tumor activity with low toxicity. GB-A-2 072 164 describes 2'-deoxy-3',5'-di-O-alkylcarbonyl-5-fluorouridine derivatives which are useful as anti-tumor agents.

The 5-fluorouracil derivatives of the invention are novel compounds undisclosed in literature and having antitumor action.

The inventor has carried out research in an attempt to improve the antitumor action of known 5-fluorouracils, 2'-deoxy-5-fluorouridines and related compounds and to render them less toxic, and consequently succeeded in synthesizing a class of novel 5-fluorouracil derivatives and 5-fluorouridine derivatives which are substituted at 3'- or 5'- position with phenyl-lower alkyloxy group optionally having specific substituent(s) and related compounds. The inventor has also found that the compounds thus prepared have an excellent antitumor action and are very useful as antitumor agents.

The present invention provides a compound represented by the formula 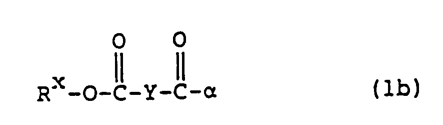
wherein R^{x} is a pyridyl group optionally having 1 to 4 substituents selected from the group consisting of hydroxy group, oxo group, halogen atom, amino group, carboxyl group, cyano group, nitro group, carbamoyl group, lower alkylcarbamoyl group, carboxy-lower alkylcarbamoyl group, phenyl-carbamoyl optionally substituted with 1 to 3 substituents selected from the group consisting of halogen atom, lower alkoxy group and lower alkyl group on the phenyl ring, lower alkoxycarbonyl-lower alkylcarbamoyl group, lower alkyl group, lower alkenyl group, lower alkoxycarbonyl group, tetrahydrofuranyl group, tetrahydropyranyl group, lower alkoxy-lower alkyl group, lower alkylthio-lower alkyl group, phenyl-lower alkoxy-lower alkyl group, phthalidyl group and acyloxy group, Y is an arylene group, and α is a known 5-fluorouracil derivative which is linked by an ester or amide linkage to the carbonyl group to which this substituent α is attached and which is selected from a group formed from a 5-fluoruoracil derivatives represented by the formulas (1-1a) and (1-1c). 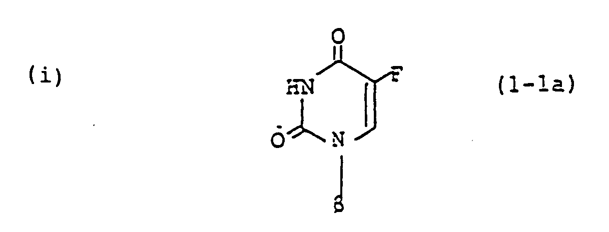
wherein β represents hydrogen atom, tetrahydrofuranyl, lower alkylcarbamoyl, phthalidyl, lower alkoxy-lower alkyl or lower alkanoyloxy-lower alkoxycarbonyl group.

The compound of the formula (1-1a) is disclosed in J. Med. Chem. 25, 1219, 1982, Cancer Chemother. Pharmacol., 1, 203-208, 1978 and Japanese Unexamined Patent Publication No. 37787/1975.

With respect to the formula (1-1a), 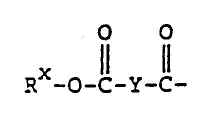
group can be linked by an amide linkage to the 3- or 1-position of the 5-fluorouracil ring. 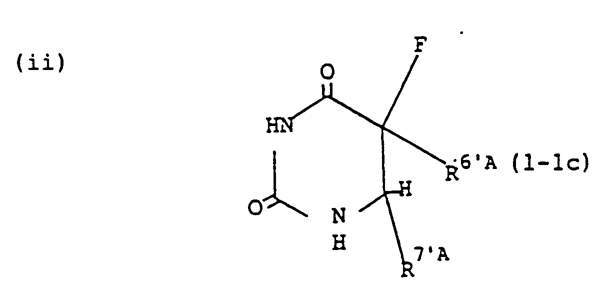
wherein R^{6'A} represents lower alkoxycarbonyl group,
R^{7'A} represents lower alkoxy group or group

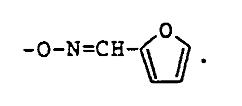

The compound of the formula (1-1c) is disclosed in EPC application published under the publication No. 99091.

Throughout the specification, the terms "lower alkyl" and "lower alkoxy" used as such or as contained in various functional groups are intended to exemplify C₁-C₆ straight or branched chain alkyl groups such as methyl, ethyl, propyl, isopropyl, butyl, tert-butyl, pentyl, hexyl, etc; and C₁-C₆ straight or branched chain alkoxy groups, such as methoxy, ethoxy, propoxy, isopropoxy, butoxy, tert-butoxy, pentyloxy, hexyloxy, etc., respectively. Further throughout the specification, the term "halogen" used as it is or as contained in various functional groups is intended to exemplify fluorine, chlorine, bromine, iodine, etc. Also throughout the specification, the term "lower alkylenedioxy" used as such or as contained in various functional groups is meant to exemplify C₁-C₄ alkylenedioxy groups such as methylenedioxy, ethylenedioxy, trimethylenedioxy, tetramethylenedioxy, etc.

With respect to the substituents for substituted phenyl-lower alkyl groups, examples of lower alkyl groups include C₁-C₆ straight or branched chain alkyl groups such as methyl, ethyl, propyl, isopropyl, butyl, tert-butyl, pentyl, hexyl, etc; examples of lower alkoxy groups are C₁-C₆ straight or branched chain alkoxy groups, such as methoxy, ethoxy, propoxy, isopropoxy, butoxy, tert-butoxy, pentyloxy, hexyloxy, etc; examples of halogen atoms are fluorine, chlorine, bromine and iodine; examples of lower alkoxycarbonyl groups are C₂-C₇ straight or branched chain alkoxycarbonyl groups such as methoxycarbonyl, ethoxycarbonyl, propoxycarbonyl, isopropoxycarbonyl, butoxycarbonyl, tert-butoxycarbonyl, pentyloxycarbonyl, hexyloxycarbonyl, etc. Examples of phenyl-lower alkyl groups optionally substituted with the above substituents or with carboxy or di(lower alkyl)amino group on the phenyl ring are phenylalkyl groups in which the phenyl group optionally substituted with one to three of the above substituents is linked with C₁-C₆ alkylene group such as methylene, ethylene, trimethylene, 1-methylethylene, tetramethylene, 2-methyltrimethylene, pentamethylene, hexamethylene, etc. Examples thereof are as follows.
benzyl, 2-methylbenzyl, 3-methylbenzyl, 4-methylbenzyl, 2-ethylbenzyl, 3-ethylbenzyl, 4-ethylbenzyl, 2-propylbenzyl, 3-propylbenzyl, 4-propylbenzyl, 2-butylbenzyl, 3-butylbenzyl, 4-butylbenzyl, 2-tert-butylbenzyl, 3-tert-butylbenzyl, 4-tert-butylbenzyl, 2-pentylbenzyl, 3-pentylbenzyl, 4-pentylbenzyl, 2-hexylbenzyl, 3-hexylbenzyl, 4-hexylbenzyl, 2,3-dimethylbenzyl, 2,4-dimethylbenzyl, 2,5-dimethylbenzyl, 2,6-dimethylbenzyl, 3,4-dimethylbenzyl, 3,5-dimethylbenzyl, 2,3,4-trimethylbenzyl, 2,4,5-trimethylbenzyl, 2,3,5-trimethylbenzyl, 2,4,6-trimethylbenzyl, 3,4,5-trimethylbenzyl, 2,3-diethylbenzyl, 2,4-diethylbenzyl, 2,5-diethylbenzyl, 2,6-diethylbenzyl, 2,4,6-triethylbenzyl, 2,4-dipropylbenzyl, 3,4,5-triethylbenzyl, 3-methyl-4-ethylbenzyl, 1-phenylethyl, 2-phenylethyl, 2-phenyl-1-methyl-ethyl, 1-(2-methylphenyl)ethyl, 2-(2-methylphenyl)ethyl, 2-(3-methylphenyl)ethyl, 2-(4-methylphenyl)ethyl, 1-(2,4-dimethylphenyl)ethyl, 2-(2,4-dimethylphenyl)ethyl, 1-(2,4,6-trimethylphenyl)ethyl, 2-(2,4,6-trimethylphenyl)ethyl, 3-phenylpropyl, 3-(4-methylphenyl)propyl, 4-phenylbutyl, 4-(2-methylphenyl)butyl, 5-phenylpentyl, 5-(3-methylphenyl)pentyl, 6-phenylhexyl, 6-(4-methylphenyl)hexyl, 2-methoxybenzyl, 3-methoxybenzyl, 4-methoxybenzyl, 2,3-dimethoxybenzyl, 2,4-dimethoxybenzyl, 2,5-dimethoxybenzyl, 3-methoxy-4-ethoxybenzyl, 2,6-dimethoxybenzyl, 3,4-dimethoxybenzyl, 3,5-dimethoxybenzyl, 2,3,4-trimethoxybenzyl, 2,4,5-trimethoxybenzyl, 2,3,5-trimethoxybenzyl, 2,4,6-trimethoxybenzyl, 3,4,5-trimethoxybenzyl, 2-ethoxybenzyl, 4-propoxybenzyl, 3-butoxybenzyl, 2-tert-butoxybenzyl, 3-pentyloxybenzyl, 4-hexyloxybenzyl, 2,3-diethoxybenzyl, 1-(4-methoxyphenyl)ethyl, 2-(3,4-dimethoxyphenyl)ethyl, 3-(4-methoxyhenyl)propyl, 4-(2-methoxyphenyl)butyl, 5-(4-methoxyphenyl)pentyl, 6-(4-methoxyphenyl)hexyl, 6-(3,4,5-tripentyloxyphenyl)hexyl, 2-fluorobenzyl, 3-fluorobenzyl, 4-fluorobenzyl, 2,3-difluorobenzyl, 2,4-difluorobenzyl, 2,5-difluorobenzyl, 2-fluoro-3-chlorobenzyl, 2-fluoro-3-bromobenzyl, 2,6-difluorobenzyl, 2,3,4-trifluorobenzyl, 2,4,5-trifluorobenzyl, 2,3,5-trifluorobenzyl, 2,4,6-trifluorobenzyl, 3,4,5-trifluorobenzyl, 1-(2-fluorophenyl)ethyl, 2-(2-fluorophenyl)ethyl, 3-(3-fluorophenyl)propyl, 4-(2-fluorophenyl)butyl, 5-(2-fluorophenyl)pentyl, 6-(3-fluorophenyl)hexyl, 2-bromobenzyl, 3-bromobenzyl, 4-bromobenzyl, 2,3-dibromobenzyl, 2-bromo-3-fluorobenzyl, 2-fluoro-4-bromobenzyl, 2,4-dibromobenzyl, 2,5-dibromobenzyl, 2,6-dibromobenzyl, 2,3,4-tribromobenzyl, 2,4,5-tribromobenzyl, 2,3,5-tribromobenzyl, 2,4,6-tribromobenzyl, 3,4,5-tribromobenzyl, 1-(2-bromophenyl)ethyl, 2-(2-bromophenyl)ethyl, 3-(2-bromophenyl)propyl, 4-(3-bromophenyl)butyl, 5-(2-bromophenyl)pentyl, 6-(4-bromophenyl)hexyl, 2-chlorobenzyl, 3-chlorobenzyl, 4-chlorobenzyl, 2,3-dichlorobenzyl, 2,4-dichlorobenzyl, 2,5-dichlorobenzyl, 2,6-dichlorobenzyl, 2-bromo-4-chlorobenzyl, 2-fluoro-4-chlorobenzyl, 2,3,4-trichlorobenzyl, 2,4,5-trichlorobenzyl, 2,3,5-trichlorobenzyl, 2,4,6-trichlorobenzyl, 3,4,5-trichlorobenzyl, 1-(4-chlorophenyl)ethyl, 2-(4-chlorophenyl)ethyl, 3-(2-chlorophenyl)propyl, 4-(4-chlorophenyl)butyl, 5-(3-chlorophenyl)pentyl, 6-(4-chlorophenyl)hexyl, 2-(3,4-dichlorophenyl)ethyl, 2-iodobenzyl, 3-iodobenzyl, 4-iodobenzyl, 3,4-diiodobenzyl, 3,4,5-triiodobenzyl, 2-(3-iodophenyl)ethyl, 6-(2-iodophenyl)hexyl, 2-carboxybenzyl, 3-carboxybenzyl, 4-carboxybenzyl, 2,3-dicarboxybenzyl, 2,4-dicarboxybenzyl, 2,5-dicarboxybenzyl, 2,6-dicarboxybenzyl, 3,4-dicarboxybenzyl, 3,5-dicarboxybenzyl, 2,3,4-tricarboxybenzyl, 2,4,6-tricarboxybenzyl, 3,4,5-tricarboxybenzyl, 1-(4-carboxyphenyl)ethyl, 2-(4-carboxyphenyl)ethyl, 2-(2,6-dicarboxyphenyl)ethyl, 3-(3-carboxyphenyl)propyl, 5-(2,4,6-tricarboxyphenyl)pentyl, 6-(4-carboxyphenyl)hexyl, 6-(3,5-dicarboxyphenyl)hexyl, 4-methoxycarbonylbenzyl, 3-methoxycarbonylbenzyl, 2-methoxycarbonylbenzyl, 4-ethoxycarbonylbenzyl, 3-ethoxycarbonylbenzyl, 2-ethoxycarbonylbenzyl, 4-propoxycarbonylbenzyl, 4-butoxycarbonylbenzyl, 4-pentyloxycarbonylbenzyl, 4-hexyloxycarbonylbenzyl, 3,4-dimethoxycarbonylbenzyl, 2,4-dimethoxycarbonylbenzyl, 3,5-dimethoxycarbonylbenzyl, 2,3,4-trimethoxycarbonylbenzyl, 3,4,5-trimethoxycarbonylbenzyl, 2-(4-methoxycarbonylphenyl)ethyl, 6-(4-methoxycarbonylphenyl)hexyl, 2-(N,N-dimethylamino)benzyl, 3-(N,N-dimethylamino)benzyl, 4-(N,N-dimethylamino)benzyl, 4-(N-methyl-N-ethylamino)benzyl, 2,4-di(N,N-dimethylamino)benzyl, 2,4,6-tri(N,N-dimethylamino)benzyl, etc.

Examples of acyl of acyloxy groups disclosed in the specification and claims and especially disclosed as the substituent of the pyridyl group represented by
R^{x} are various and include the following:
(i) C₁-C₂₀ alkanoyl groups optionally substituted with the substituents selected from the group consisting of halogen atom, hydroxy group, lower alkoxy group, aryloxy group, substituted or unsubstituted aryl group, phenyl-lower alkoxycarbonyl group and lower alkylcarbamoyl group.
(ii) arylcarbonyl groups optionally substituted with lower alkylenedioxy group or with 1 to 3 substituents selected from the group consisting of halogen atom, lower alkyl group, lower alkoxy group, carboxy group, lower alkoxycarbonyl group, nitro group, cyano group, phenyl-lower alkoxycarbonyl group, hydroxy group, guanidyl group, phenyl-lower alkoxy group, amino group and amino group substituted with lower alkyl group.
(iii) 5- or 6- membered unsaturated hetero ring-carbonyl groups having nitrogen atom, sulfur atom or oxygen atom as the hetero atom.
(iv) carbonic acid ester residue such as aryloxycarbonyl groups, straight or branched-chain or cyclic alkoxycarbonyl groups.
(v) substituted or unsubstituted cycloalkylcarbonyl groups.
(vi) lower alkenyl (or lower alkynyl)carbonyl groups. (vii) lower alkenyl (or lower alkynyl)oxycarbonyl groups.

Examples of the acyl moieties included in the items (i) to (vii) are as follows.
(i) C₁-C₂₀ alkanoyl groups optionally substituted with the substituents selected from the group consisting of halogen atom, hydroxy group, lower alkoxy group, aryloxy group, substituted or unsubstituted aryl group, phenyl-lower alkoxycarbonyl group and lower alkylcarbamoyl group.
   C₁-C₂₀ unsubstituted alkanoyl groups such as formyl, acetyl, propionyl, butyryl, isobutyryl, pentanoyl, hexanoyl, heptanoyl, octanoyl, nonanoyl, decanoyl, undecanoyl, dodecanoyl, tridecanoyl, tetradecanoyl, pentadecanoyl, hexadecanoyl, heptadecanoyl, ocatadecanoyl, nonadecanoyl, eicosanoyl, etc; C₂-C₆ alkanoyl groups substituted with 1 to 3 halogen atoms such as monochloroacetyl, monobromoacetyl, dichloroacetyl, trichloroacetyl, 3-chloropropionyl, 4-chlorobutyryl, 5-chloropentanoyl, 6-chlorohexanoyl, etc; C₂-C₆ alkanoyl groups substituted with hydroxy group such as hydroxyacetyl, 3-hydroxypropionyl, 5-hydroxypentanoyl, 4-hydroxybutanoyl, 6-hydroxyhexanoyl, etc; C₂-C₆ alkanoyl groups substituted with C₁-C₆ alkoxy group such as methoxyacetyl, ethoxyacetyl, 3-propoxypropionyl, 6-hexyloxyhexanoyl, 3-methoxypropionyl, etc; C₂-C₆ alkanoyl groups substituted with phenoxy or naphthyloxy group such as phenoxyacetyl, 2-phenoxypropionyl, 3-phenoxypropionyl, 4-phenoxybutyryl, 5-phenoxypentanoyl, 6-phenoxyhexanoyl, α-naphthyloxyacetyl, etc; C₂-C₆ alkanoyl groups substituted with aryl group optionally substituted with 1 to 3 substituents selected from the group consisting of halogen atom, lower alkyl group, lower alkoxy group, carboxy group, lower alkoxycarbonyl group, nitro group and cyano group on the aryl ring (phenyl ring, naphthyl ring, etc.), such as α-phenylacetyl, 2-phenylpropionyl, 3-phenylpropionyl, 4-phenylbutyryl, 5-phenylpentanoyl, 6-phenylhexanoyl, α-(2-chlorophenyl)acetyl, α-(4-methylphenyl)acetyl, α-(3,4,5-trimethoxyphenyl)acetyl, α-(3,4-dimethoxyphenyl)acetyl, 6-(4-carboxyphenyl)hexanoyl, 4-(4-ethoxycarbonylphenyl)pentanoyl, α-(4-nitrophenyl)acetyl, α-(4-cyanophenyl)acetyl, α-naphthylacetyl, β-naphthylacetyl, etc; C₁-C₂₀ alkanoyl groups substituted with phenyl-C₂-C₇ lower alkoxycarbonyl group such as α-benzyloxycarbonylacetyl, 2-benzyloxycarbonylpropionyl, 3-benzyloxycarbonylpropionyl, 4-benzyloxycarbonylbutyryl, 5-benzyloxycarbonylpentanoyl, 6-benzyloxycarbonylhexanoyl, 3-(α-phenethyloxycarbonyl)propionyl, 3-(β-phenethyloxycarbonyl)propionyl, 5-(benzyloxycarbonyl)hexanoyl, 7-(benzyloxycarbonyl)heptanoyl, 8-(α-phenethyloxycarbonyl)octanoyl, 9-(β-phenethyloxycarbonyl)nonanoyl, 10-(benzyloxycarbonyl)decanoyl, 11-(β-phenethyloxycarbonyl)tridecanoyl, 15-(benzyloxycarbonyl)pentadecanoyl, 17-(benzyloxycarbonyl)heptadecanoyl, 20-(benzyloxycarbonyl)eicosanoyl, etc; C₁-C₂₀ alkanoyl groups substituted with C₂-C₇ lower alkylcarbamoyl group such as methylcarbamoylacetyl, ethylcarbamoylacetyl, propylcarbamoylacetyl, butylcarbamoylacetyl, tert-butylcarbamoylacetyl, pentylcarbamoylacetyl, hexylcarbamoylacetyl, methylcarbamoylpropionyl, ethylcarbamoylbutyryl, propylcarbamoylpentanoyl, ethylcarbamoylhexanoyl, ethylcarbamoylheptanoyl, methylcarbamoyloctanoyl, ethylcarbamoylnonanoyl, methylcarbamoyldecanoyl, methylcarbamoytridecanoyl, ethylcarbamoylpentadecanoyl, methylcarbamoylheptadecanoyl, methylcarbamoyleicosaonyl, etc;
(ii) aryl-carbonyl groups optionally substituted with lower alkylenedioxy group or with 1 to 3 substituents selected from the group consisting of halogen atom, lower alkyl group, lower alkoxy group, carboxy group, lower alkoxycarbonyl group, nitro group, cyano group, phenyl-lower alkoxycarboxy group, hydroxy group, guanidyl group, phenyl-lower alkoxy group, amino group and amino group substituted with lower alkyl group on the aryl ring.
   aryl-carbonyl groups such as phenylcarbonyl, naphthylcarbonyl, etc. optionally substituted with lower alkylenedioxy group or with 1 to 3 substituents selected from the group consisting of halogen atom, lower alkyl group, lower alkoxy group, carboxy group, lower alkoxycarbonyl groups, nitro group, cyano group, phenyl-lower alkoxycarbonyl group, hydroxy group, guanidyl group, phenyl-lower alkoxy group, amino group and amino group substituted with lower alkyl group, such as benzoyl, α-naphthylcarbonyl, β-naphthylcarbonyl, 2-methylbenzoyl, 3-methylbenzoyl, 4-methylbenzoyl, 2,4-dimethylbenzoyl, 3,4,5-trimethylbenzoyl, 4-ethylbenzoyl, 2-methoxybenzoyl, 3-methoxybenzoyl, 4-methoxybenzoyl, 2,4-dimethoxybenzoyl, 3,4,5-trimethoxybenzoyl, 4-ethoxybenzoyl, 2-methoxy-4-ethoxybenzoyl, 2-propoxybenzoyl, 3-propoxybenzoyl, 4-propoxybenzoyl, 2,4-dipropoxybenzoyl, 3,4,5-tripropoxybenzoyl, 2-carboxybenzoyl, 3-carboxybenzoyl, 4-carboxybenzoyl, 2-chlorobenzoyl, 3-chlorobenzoyl, 4-chlorobenzoyl, 2,3-dichlorobenzoyl, 2,4,6-trichlorobenzoyl, 2-bromobenzoyl, 4-fluorobenzoyl, 2-methoxycarbonylbenzoyl, 3-methoxycarbonylbenzoyl, 4-methoxycarbonylbenzoyl, 2-ethoxycarbonylbenzoyl, 3-ethoxycarbonylbenzoyl, 4-ethoxycarbonylbenzoyl, 2-propoxycarbonylbenzoyl, 3-propoxycarbonylbenzoyl, 4-propoxycarbonylbenzoyl, 2-isopropoxycarbonylbenzoyl, 3-isopropoxycarbonylbenzoyl, 4-isopropoxycarbonylbenzoyl, 2-butoxycarbonylbenzoyl, 3-butoxycarbonylbenzoyl, 4-butoxycarbonylbenzoyl, 2-tert-butoxycarbonylbenzoyl, 3-tert-butoxycarbonylbenzoyl, 4-tert-butoxycarbonylbenzoyl, 2-pentyloxycarbonylbenzoyl, 3-pentyloxycarbonylbenzoyl, 4-pentyloxycarbonylbenzoyl, 2-hexyloxycarbonylbenzoyl, 3-hexyloxycarbonylbenzoyl, 4-hexyloxycarbonylbenzoyl, 3,5-dimethoxycarbonylbenzoyl, 3,4,5-trimethoxycarbonylbenzoyl, β-methyl-α-naphthylcarbonyl, α-methoxy-β-naphthylcarbonyl, β-chloro-α-naphthylcarbonyl, 2-cyanobenzoyl, 4-cyanobenzoyl, 2-nitrobenzoyl, 4-nitrobenzoyl, 2-benzyloxycarbonylbenzoyl, 3-benzyloxycarbonylbenzoyl, 4-benzyloxycarbonylbenzoyl, 3-(α-phenethyloxycarbonyl)benzoyl, 4-(β-phenethyloxycarbonyl)benzoyl, 4-(3-phenylpropoxycarbonyl)benzoyl, 4-(6-phenylhexyloxycarbonyl)benzoyl, 2-hydroxybenzoyl, 3-hydroxybenzoyl, 2,3-dihydroxybenzoyl, 3,4-dihydroxybenzoyl, 3,4,5-trihydroxybenzoyl, 4-hydroxybenzoyl, 2-guanidylbenzoyl, 3-guanidylbenzoyl, 4-guanidylbenzoyl, 2-(benzyloxy)benzoyl, 3-(benzyloxy)benzoyl, 4-(benzyloxy)benzoyl, 2-(α-phenethyloxy)benzoyl, 3-(α-phenethyloxy)benzoyl, 4-(α-phenethyloxy)benzoyl, 2-(β-phenethyloxy)benzoyl, 3-(β-phenethyloxy)benzoyl, 4-(β-phenethyloxy)benzoyl, 4-(3-phenylpropoxy)benzoyl, 4-(4-phenylbutoxy)benzoyl, 4-(5-phenylpentyloxy)benzoyl, 4-(6-phenylhexyloxy)benzoyl, 2-aminobenzoyl, 3-aminobenzoyl, 4-aminobenzoyl, 2-methylaminobenzoyl, 3-methylaminobenzoyl, 4-methylaminobenzoyl, 2-ethylaminobenzoyl, 3-propylaminobenzoyl, 4-butylaminobenzoyl, 3-pentylaminobenzoyl, 4-hexylaminobenzoyl, 2-(N,N-dimethylamino)benzoyl, 3-(N,N-dimethylamino)benzoyl, 4-(N,N-dimethylamino)benzoyl, 3-(N-methyl-N-ethylamino)benzoyl, 4-(N,N-diethylamino)benzoyl, 3-(N,N-dipropylamino)benzoyl, 4-(N,N-dibutylamino)benzoyl, 4-(N,N-dipentylamino)benzoyl, 4-(N,N-dihexylamino)benzoyl, 3-benzyloxycarbonyl-1-naphthylcarbonyl, 6-(β-phenethyloxycarbonyl)-1-naphthylcarbonyl, 4-benzyloxycarbonyl-2-naphthylcarbonyl, 5-(α-phenethyloxycarbonyl)-2-naphthylcarbonyl, 2-hydroxy-1-naphthylcarbonyl, 3-hydroxy-1-naphthylcarbonyl, 4-hydroxy-1-naphthylcarbonyl, 5-hydroxy-1-naphthylcarbonyl, 6-hydroxy-1-naphthylcarbonyl, 7-hydroxy-1-naphtylcarbonyl, 8-hydroxy-1-naphthylcarbonyl, 1-hydroxy-2-naphthylcarbonyl, 4-hydroxy-2-naphthylcarbonyl, 5-hydroxy-2-naphthylcarbonyl, 7-hydroxy-2-naphthylcarbonyl, 2-guanidyl-1-naphtylcarbonyl, 3-guanidyl-1-naphthylcarbonyl, 5-guanidyl-1-naphthylcarbonyl, 6-guanidyl-1-naphthylcarbonyl, 8-guanidyl-1-naphthylcarbonyl, 1-guanidyl-2-naphthylcarbonyl, 4-guanidyl-2-naphthylcarbonyl, 6-guanidyl-2-naphthylcarbonyl, 8-guanidyl-2-naphthylcarbonyl, 2-amino-1-naphthylcarbonyl, 3-amino-1-naphthylcarbonyl, 4-amino-1-naphthylcarbonyl, 6-amino-1-naphthylcarbonyl, 4-amino-2-naphthylcarbonyl, 5-amino-1-naphthylcarbonyl, 7-amino-2-naphthylcarbonyl, 8-amino-2-naphthylcarbonyl, 3-(N,N-dimethylamino)-2-naphthylcarbonyl, 4-(N-methyl-N-ethylamino)-1-naphthylcarbonyl, 6-(N,N-dimethylamino)-1-naphthylcarbonyl, 7-(N-methyl-N-ethylamino)-2-naphthylcarbonyl, 8-(N-methyl-N-ethylamino)-1-naphthylcarbonyl, 3,4-methylenedioxybenzoyl, 3,4-ethylenedioxybenzoyl, 2,3-methylenedioxybenzoyl, etc.
(iii) 5- or 6- membered unsaturated hetero ring-carbonyl groups having nitrogen atom, sulfur atom or oxygen atom as the hetero atom.
   thienylcarbonyl, furanylcarbonyl, thiazolylcarbonyl, quinolylcarbonyl, pyrazinylcarbonyl, pyridylcarbonyl, etc., such as 2-thienylcarbonyl, 3-thienylcarbonyl, 2-furanylcarbonyl, 3-furanylcarbonyl, 4-thiazolylcarbonyl, 2-quinolylcarbonyl, 2-pyrazinylcarbonyl, 2-pyridylcarbonyl, 3-pyridylcarbonyl, 4-pyridylcarbonyl, etc.
(iv) carbonic acid ester residue such as aryloxycarbonyl groups, straight or branched-chain or cyclic alkoxycarbonyl groups.
   aryloxycarbonyl groups optionally substituted with 1 to 3 substituents selected from the group consisting of halogen atom, lower alkyl group and lower alkoxy group on the aryl ring (phenyl ring, naphthyl ring, etc.), such as phenoxycarbonyl, α-naphthyloxycarbonyl, β-naphthyloxycarbonyl, 2-methylphenoxycarbonyl, 3-methylphenoxycarbonyl, 4-methylphenoxycarbonyl, 2,4-dimethylphenoxycarbonyl, 3,4,5-trimethylphenoxycarbonyl, 4-ethylphenoxycarbonyl, 2-methoxyphenoxycarbonyl, 3-methoxyphenoxycarbonyl, 4-methoxyphenoxycarbonyl, 2,4-dimethoxyphenoxycarbonyl, 3,4,5-trimethoxyphenoxycarbonyl, 4-ethoxyphenoxycarbonyl, 2-propoxyphenoxycarbonyl, 3-propoxyphenoxycarbonyl, 4-propoxyphenoxycarbonyl, 2,4-dipropoxyphenoxycarbonyl, 3,4,5-tripropoxyphenoxycarbonyl, 2-chlorophenoxycarbonyl, 3-chlorophenoxycarbonyl, 4-chlorophenoxycarbonyl, 2,3-dichlorophenoxycarbonyl, 2,4,6-trichlorophenoxycarbonyl, 2-bromophenoxycarbonyl, 4-fluorophenoxycarbonyl, β-methyl-α-naphthyloxycarbonyl, α-methoxy-β-naphthyloxycarbonyl, β-chloro-α-naphthyloxycarbonyl etc; straight or branched-chain or cyclic alkoxycarbonyl groups having 1 to 8 carbon atoms in the alkoxy moiety, such as methoxycarbonyl, ethoxycarbonyl, propoxycarbonyl, isopropoxycarbonyl, tert-butoxycarbonyl, butoxycarbonyl, pentyloxycarbonyl, hexyloxycarbonyl, cyclopropyloxycarbonyl, cyclobutyloxycarbonyl, cyclopentyloxycarbonyl, cyclohexyloxycarbonyl, cycloheptyloxycarbonyl, cyclooctyloxycarbonyl, etc.
(v) substituted or unsubstituted cycloalkyl carbonyl groups.
   cycloalkylcarbonyl groups optionally substituted with halogen atom, hydroxy group, lower alkoxy group or lower alkyl group and having 3 to 8 carbon atoms in the cycloalkyl ring, such as cyclopropylcarbonyl, cyclobutylcarbonyl, cyclopentylcarbonyl, cyclohexylcarbonyl, cycloheptylcarbonyl, cyclooctylcarbonyl, 2-chlorocyclohexylcarbonyl, 3-hydroxycyclopentylcarbonyl, 3-methylcyclohexylcarbonyl, 4-methoxycyclohexylcarbonyl, etc.
(vi) lower alkenyl (or lower alkynyl)carbonyl groups.
   carbonyl groups having C₂-C₆ alkenyl or alkynyl group, such as vinylcarbonyl, allylcarbonyl, 2-butenylcarbonyl, 3-butenylcarbonyl, 1-methylallylcarbonyl, 2-pentenylcarbonyl, 3-hexenylcarbonyl, ethynylcarbonyl, propynylcarbonyl, 2-butynylcarbonyl, 1-methyl-3-pentynylcarbonyl, 4-hexynylcarbonyl, etc.
(vii) lower alkenyl (or lower alkynyl)oxycarbonyl groups.
   carbonyl groups having C₂-C₆ alkenyloxy or alkynyloxy group, such as vinyloxycarbonyl, allyloxycarbonyl, 2-butenyloxycarbonyl, 3-butenyloxycarbonyl, 2-hexenyloxycarbonyl, 1-methylallyloxycarbonyl, 2-pentenyloxycarbonyl, 3-hexenyloxycarbonyl, ethynyloxycarbonyl, propynyloxycarbonyl, 2-butynyloxycarbonyl, 1-methyl-3-pentynyloxycarbonyl; 4-hexynyloxycarbonyl, etc.

The substituents on the substituted pyridyl group represented by R^{x} are exemplified below.

Examples of lower alkylcarbamoyl groups are alkylcarbamoyl groups having one or two C₁-C₆ alkyl groups such as N-methylcarbamoyl, N-ethylcarbamoyl, N-propylcarbamoyl, N-isopropylcarbamoyl, N-butylcarbamoyl, N-tert-butylcarbamoyl, N-pentylcarbamoyl, N-hexylcarbamoyl, N,N-dimethylcarbamoyl, N,N-diethylcarbamoyl, N,N-dipropylcarbamoyl, N-isopropyl-N-methylcarbamoyl, N-ethyl-N-methylcarbamoyl, N-methyl-N-pentylcarbamoyl, N-propyl-N-pentylcarbamoyl, N,N-dipentylcarbamoyl, N-ethyl-N-hexylcarbamoyl, N-hexyl-N-pentylcarbamoyl, N,N-dihexylcarbamoyl and the like.

Examples of phenylcarbamoyl groups optionally having 1 to 3 substituents selected from the group consisting of halogen, lower alkyl and lower alkoxy on the phenyl ring are carbamoyl groups having one or two phenyl groups which may optionally have 1 to 3 substituents selected from the group consisting of halogen, C₁-C₆ alkoxy and C₁-C₆ alkyl on the phenyl ring such as N-(2-chlorophenyl)carbamoyl, N-(3,5-dichloro)phenylcarbamoyl, N-(3-methoxyphenyl)carbamoyl, N-(4-propoxyphenyl)carbamoyl, N-(2-methylphenyl)carbamoyl, N-(4-ethylphenyl)carbamoyl, N-(3-isopropylphenyl)carbamoyl, N-(4-hexylphenyl)carbamoyl, N-phenylcarbamoyl, N,N-diphenylcarbanoyl and the like.

Examples of lower alkenyl groups are C₂-C₆ alkenyl groups such as vinyl, allyl, 2-butenyl, 3-butenyl, 1-methylallyl, 2-pentenyl, 2-hexenyl and the like.

Examples of lower alkoxycarbonyl groups are carbonyl groups having C₁-C₆ alkoxy group such as methoxycarbonyl, ethoxycarbonyl, propoxycarbonyl, isopropoxycarbonyl, tert-butoxycarbonyl, butoxycarbonyl, pentyloxycarbonyl, hexyloxycarbonyl and the like.

Examples of tetrahydrofuranyl groups are 2-tetrahydrofuranyl, 3-tetrahydrofuranyl and the like.

Examples of lower alkoxy-lower alkyl groups are alkoxyalkyl groups in which the alkoxy moiety and alkyl moiety each have 1 to 6 carbon atoms, such as methoxymethyl, 3-methoxypropyl, 4-ethoxybutyl, 6-propoxyhexyl, 5-isopropoxypentyl, 1,1-dimethyl-2-butoxyethyl, 2-methyl-3-tert-butoxypropyl, 2-pentyloxyethyl, 2-hexyloxyethyl and the like.

Examples of phenyl-lower alkoxy-lower alkyl groups are alkoxyalkyl groups which is substituted with phenyl group and in which the alkoxy moiety and alkyl moiety each have 1 to 6 carbon atoms, such as benzyloxymethyl, 2-benzyloxyethyl, 5-(2-phenylpentyloxy)pentyl, 6-benzyloxyhexyl, 4-hexyloxyhexyl, phenylmethoxymethyl, 2-phenylethoxymethyl, 2-(phenylmethoxy)ethyl, 2-(phenylmethoxy)propyl, 4-(phenylmethoxy)butyl, 5-(phenylmethoxy)pentyl, 6-(phenylmethoxy)hexyl, 2-(2-phenylethoxy)ethyl, 2-(4-phenylbutoxy)ethyl, 4-(4-phenylbutoxy)butyl, 6-phenylmethoxyhexyl and the like.

Examples of lower alkoxycarbonyl-lower alkylcarbamoyl groups are carbamoyl groups substituted with one alkoxycarbonylalkyl group in which the alkoxy moiety and the alkyl moiety each have 1 to 6 carbon atoms, such as methoxycarbonylmethylcarbamoyl, 3-methoxycarbonylpropylcarbamoyl, ethoxycarbonylmethylcarbamoyl, propoxycarbonylmethylcarbamoyl, isopropoxycarbonylmethylcarbamoyl, butoxycarbonylmethylcarbamoyl, tert-butoxycarbonylmethylcarbamoyl, pentyloxycarbonylmethylcarbamoyl, hexyloxycarbonylmethylcarbamoyl, 2-methoxycarbonylethylcarbamoyl, 2-methoxycarbonyl-1-methylethylcarbamoyl, 4-methoxycarbonylbutylcarbamoyl, 2-methoxycarbonyl-1,1-dimethylethylcarbamoyl, 5-methoxycarbonylpentylcarbamoyl, 6-methoxycarbonylhexylcarbamoyl, 2-ethoxycarbonylethylcarbamoyl, 4-ethoxycarbonylbutylcarbamoyl, 6-propoxycarbonylhexylcarbamoyl, 5-isopropoxycarbonylpentylcarbamoyl, 1,1-dimethyl-2-butoxycarbonylethylcarbamoyl, 2-methyl-3-tert-butoxycarbonylpropylcarbamoyl, 2-pentyloxycarbonylethylcarbamoyl, hexyloxycarbonylethylcarbamoyl and the like.

Examples of carboxy-lower alkylcarbamoyl groups are carboxy-C₁-C₆ alkylcarbamoyl groups, such as N-(carboxymethyl)carbamoyl, N-(2-carboxyethyl)carbamoyl, N-(3-carboxypropyl)carbamoyl, N-(2-methyl-2-carboxyethyl)carbamoyl, N-(4-carboxybutyl)carbamoyl, N-(2-methyl-3-carboxypropyl)carbamoyl, N-(2,2-dimethyl-2-carboxyethyl)carbamoyl, N-(5-carboxypentyl)carbamoyl, N-(6-carboxyhexyl)carbamoyl and the like.

Examples of tetrahydropyranyl groups are 2-tetrahydropyranyl, 3-tetrahydropyranyl, 4-tetrahydropyranyl and the like.

Examples of lower alkylthio-lower alkyl groups are C₁-C₆ alkylthio-C₁-C₆ alkyl groups, such as methylthiomethyl, ethylthiomethyl, propylthiomethyl, butylthiomethyl, tert-butylthiomethyl, pentylthiomethyl, hexylthiomethyl, methylthioethyl, methylthiopropyl, methylthiobutyl, methylthiopentyl, methylthiohexyl, ethylthioethyl, ethylthiobutyl, propylthiohexyl and the like.

The pyridine ring is preferably substituted at any of the 2- to 6-positions with one to four of the substituents selected from the group consisting of the hydroxy, halogen, amino, carboxyl, cyano, nitro, oxo, lower alkyl, lower alkenyl, lower alkoxycarbonyl, carbamoyl and acyloxy among the substituents exemplified above, or preferably substituted at the 1-position with the carbamoyl, lower alkylcarbamoyl, phenylcarbamoyl optionally having 1 to 3 substituents selected from the group consisting of halogen, lower alkoxy and lower alkyl on the phenyl ring, tetrahydrofuranyl, phthalidyl, lower alkoxy-lower alkyl, phenyl-lower alkoxy-lower alkyl or lower alkoxycarbonyl-lower alkylcarbamoyl group among the substituents exemplified above.
Preferable examples of the group of the formula (Y), i.e., 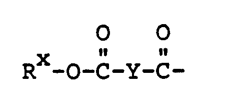
are those represented by the formula 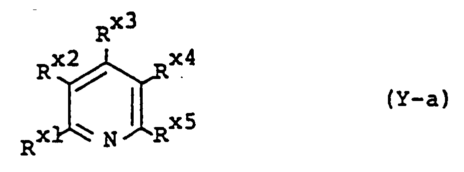
wherein R^{x1} is hydroxy or acyloxy; R^{x2} and R^{x4} are each hydrogen, halogen, amino, carboxyl, carbamoyl, cyano, nitro, lower alkyl, lower alkenyl or lower alkoxycarbonyl; R^{x3} and R^{x5} are each hydrogen, hydroxy or acyloxy; when at least one of R^{x1}, R^{x3} and R^{x5} is free hydroxy, the structure of 1-position on the pyridine ring can be 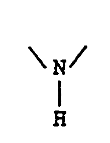
due to the keto-enol tautomerism, said hydrogen attached to nitrogen being optionally substituted with a substituent selected from the group consisting of lower alkyl, tetrahydrofuranyl, tetrahydropyranyl, lower alkoxy-lower alkyl, phthalidyl, carbamoyl, lower alkoxycarbonyl-lower alkylcarbamoyl, phenyl-lower alkoxy-lower alkyl, phenylcarbamoyl which may have 1 to 3 substituents selected from the group consisting of halogen, lower alkoxy and lower alkyl on the phenyl ring, lower alkylcarbamoyl, carboxy-lower alkylcarbamoyl, lower alkylthio-lower alkyl and lower alkenyl, provided that at least one of R^{x1}, R^{x3} and R^{x5} represents a hydroxy group and that the hydrogen of one hydroxyl group represented by R^{x1}, R^{x3} or R^{x5} is substituted with a group 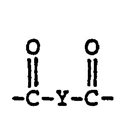
wherein Y is as defined above.

More preferable groups of the formula (Y) are those represented by the formula (Y-a) wherein R^{x4} is halogen, amino, carboxyl, carbamoyl, cyano, nitro, lower alkyl, lower alkenyl or lower alkoxycarbonyl.

The most preferable groups of the formula (Y) are those represented by the formula (Y-a) wherein R^{x4} is halogen or cyano.

Examples of the pyridyl groups represented by R^{x} and optionally having the above various substituents are as follows.
2-pyridyl, 3-pyridyl, 4-pyridyl, 2-hydroxy-3-pyridyl, 2-hydroxy-4-pyridyl, 2-hydroxy-5-pyridyl, 2-hydroxy-6-pyridyl, 2-hydroxy-5-chloro-3-pyridyl, 2-hydroxy-5-chloro-4-pyridyl, 4-hydroxy-5-chloro-2-pyridyl, 2-hydroxy-5-chloro-6-pyridyl, 2-hydroxy-5-fluoro-4-pyridyl, 4-hydroxy-5-fluoro-2-pyridyl, 2-hydroxy-5-fluoro-6-pyridyl, 2-hydroxy-5-bromo-4-pyridyl, 4-hydroxy-5-bromo-2-pyridyl, 2-hydroxy-5-bromo-6-pyridyl, 2-hydroxy-5-iodo-4-pyridyl, 4-hydroxy-5-iodo-2-pyridyl, 2-hydroxy-5-iodo-6-pyridyl, 2-hydroxy-3-bromo-4-pyridyl, 4-hydroxy-3-bromo-2-pyridyl, 2-hydroxy-3-chloro-4-pyridyl, 4-hydroxy-3-chloro-2-pyridyl, 2-hydroxy-3-chloro-6-pyridyl, 2-hydroxy-4-chloro-6-pyridyl, 1-(2-tetrahydrofuranyl)-1,2-dihydro-2-oxo-3-pyridyl, 1-(2-tetrahydrofuranyl)-1,2-dihydro-2-oxo-4-pyridyl, 1-(2-tetrahydrofuranyl)-1,2-dihydro-2-oxo-5-pyridyl, 1-(2-tetrahydrofuranyl)-1,2-dihydro-2-oxo-6-pyridyl, 1-(3-tetrahydrofuranyl)-1,2-dihydro-2-oxo-4-pyridyl, 1-(2-tetrahydrofuranyl)-1,2-dihydro-2-oxo-5-chloro-4-pyridyl, 1-(2-tetrahydrofuranyl)-1,2-dihydro-2-oxo-5-chloro-6-pyridyl, 1-(2-tetrahydrofuranyl)-1,2-dihydro-2-oxo-5-fluoro-4-pyridyl, 1-(2-tetrahydrofuranyl)-1,2-dihydro-2-oxo-5-bromo-4-pyridyl, 1-(2-tetrahydrofuranyl)-1,2-dihydro-2-oxo-5-iodo-4-pyridyl, 1-(3-tetrahydrofuranyl)-1,2-dihydro-2-oxo-5-chloro-4-pyridyl, 1-(2-tetrahydrofuranyl)-1,2-dihydro-2-oxo-3-chloro-4-pyridyl, 1-ethoxymethyl-1,2-dihydro-2-oxo-3-pyridyl, 1-ethoxymethyl-1,2-dihydro-2-oxo-4-pyridyl, 1-ethoxymethyl-1,2-dihydro-2-oxo-5-pyridyl, 1-ethoxymethyl-1,2-dihydro-2-oxo-6-pyridyl, 1-methoxymethyl-1,2-dihydro-2-oxo-4-pyridyl, 1-(2-methoxyethyl)-1,2-dihydro-2-oxo-4-pyridyl, 1-ethoxymethyl-1,2-dihydro-2-oxo-5-chloro-3-pyridyl, 1-ethoxymethyl-1,2-dihydro-2-oxo-5-chloro-4-pyridyl, 1-ethoxymethyl-1,2-dihydro-2-oxo-5-chloro-6-pyridyl, 1-ethoxymethyl-1,2-dihydro-2-oxo-5-fluoro-4-pyridyl, 1-ethoxymethyl-1,2-dihydro-2-oxo-5-bromo-4-pyridyl, 1-ethoxymethyl-1,2-dihydro-2-oxo-5-iodo-4-pyridyl, 1-methoxymethyl-1,2-dihydro-2-oxo-5-chloro-4-pyridyl, 1-(2-methoxyethyl)-1,2-dihydro-2-oxo-5-chloro-4-pyridyl, 1-(2-ethoxymethyl)-1,2-dihydro-2-oxo-3-chloro-4-pyridyl, 2-acetyloxy-5-chloro-4-pyridyl, 2-acetyloxy-5-fluoro-4-pyridyl, 2-acetyloxy-5-bromo-4-pyridyl, 2-acetyloxy-5-iodo-4-pyridyl, 2-acetyloxy-5-methyl-4-pyridyl, 2-acetyloxy-5-cyano-4-pyridyl, 2-acetyloxy-5-nitro-4-pyridyl, 2-acetyloxy-5-carboxy-4-pyridyl, 2-propanoyloxy-5-chloro-4-pyridyl, 2-butanoyloxy-5-chloro-4-pyridyl, 2-isobutanoyloxy-5-chloro-4-pyridyl, 2-pentanoyloxy-5-chloro-4-pyridyl, 2-hexanoyloxy-5-chloro-4-pyridyl, 3-acetyloxy-5-chloro-4-pyridyl, 2-acetyloxy-3-chloro-4-pyridyl, 4-acetyloxy-5-chloro-2-pyridyl, 4-acetyloxy-5-fluoro-2-pyridyl, 4-acetyloxy-5-bromo-2-pyridyl, 4-acetyloxy-5-iodo-2-pyridyl, 4-acetyloxy-5-methyl-2-pyridyl, 4-acetyloxy-5-cyano-2-pyridyl, 4-acetyloxy-5-nitro-2-pyridyl, 4-acetyloxy-5-carboxy-2-pyridyl, 6-acetyloxy-5-chloro-2-pyridyl, 4-propanoyloxy-5-chloro-2-pyridyl, 4-butanoyloxy-5-chloro-2-pyridyl, 4-isobutanoyloxy-5-chloro-2-pyridyl, 4-pentanoyloxy-5-chloro-2-pyridyl, 4-hexanoyloxy-5-chloro-2-pyridyl, 4-acetyloxy-3-chloro-2-pyridyl, 3-acetyloxy-5-chloro-2-pyridyl, 2-acetyloxy-4-pyridyl, 2-propanoyloxy-4-pyridyl, 2-hexanoyloxy-4-pyridyl, 4-acetyloxy-2-pyridyl, 4-propanoyloxy-2-pyridyl, 4-hexanoyloxy-2-pyridyl, 2-methoxycarbonyloxy-5-chloro-4-pyridyl, 2-methoxycarbonyloxy-6-chloro-4-pyridyl, 2-ethoxycarbonyloxy-3-chloro-4-pyridyl, 2-ethoxycarbonyloxy-5-chloro-4-pyridyl, 2-ethoxycarbonyloxy-5-fluoro-4-pyridyl, 2-ethoxycarbonyloxy-5-bromo-4-pyridyl, 2-ethoxycarbonyloxy-6-chloro-4-pyridyl, 2-ethoxycarbonyloxy-5-chloro-3-pyridyl, 2-ethoxycarbonyloxy-5-chloro-6-pyridyl, 2-propoxycarbonyloxy-5-chloro-4-pyridyl, 2-hexyloxycarbonyloxy-5-chloro-4-pyridyl, 2-benzoyloxy-4-pyridyl, 3-benzoyloxy-4-pyridyl, 4-benzoyloxy-2-pyridyl, 3-benzoyloxy-2-pyridyl, 2-(2-methylbenzoyl)oxy-4-pyridyl, 2-(3-methylbenzoyl)oxy-4-pyridyl, 2-(4-methylbenzoyl)oxy-4-pyridyl, 2-(4-ethylbenzoyl)oxy-4-pyridyl, 2-(2-chlorobenzoyl)oxy-4-pyridyl, 2-(3-chlorobenzoyl)oxy-4-pyridyl, 2-(4-chlorobenzoyl)oxy-4-pyridyl, 2-(4-fluorobenzoyl)oxy-4-pyridyl, 2-(4-tert-butylbenzoyl)oxy-4-pyridyl, 2-(4-hexylbenzoyl)oxy-4-pyridyl, 4-(2-methylbenzoyl)oxy-2-pyridyl, 4-(4-ethylbenzoyl)oxy-2-pyridyl, 4-(3-chlorobenzoyl)oxy-2-pyridyl, 4-(4-fluorobenzoyl)oxy-2-pyridyl, 4-(4-tert-butylbenzoyl)oxy-2-pyridyl, 2-benzoyloxy-5-chloro-4-pyridyl, 2-benzoyloxy-5-fluoro-4-pyridyl, 2-benzoyloxy-5-bromo-4-pyridyl, 2-benzoyloxy-5-iodo-4-pyridyl, 2-benzoyloxy-5-methyl-4-pyridyl, 2-benzoyloxy-5-cyano-4-pyridyl, 2-benzoyloxy-5-nitro-4-pyridyl, 2-benzoyloxy-5-carboxy-4-pyridyl, 3-benzoyloxy-5-chloro-4-pyridyl, 2-benzoyloxy-3-chloro-4-pyridyl, 2-(2-methylbenzoyl)oxy-5-chloro-4-pyridyl, 2-(3-methylbenzoyl)oxy-5-chloro-4-pyridyl, 2-(4-methylbenzoyl)oxy-5-chloro-4-pyridyl, 2-(3,4,5-trimethylbenzoyl)oxy-5-chloro-4-pyridyl, 2-(4-ethylbenzoyl)oxy-5-chloro-4-pyridyl, 2-(4-tert-butylbenzoyl)oxy-5-fluoro-4-pyridyl, 2-(4-hexylbenzoyl)oxy-5-bromo-4-pyridyl, 2-(2-chlorobenzoyl)oxy-5-chloro-4-pyridyl, 2-(3-chlorobenzoyl)oxy-5-chloro-4-pyridyl, 2-(4-chlorobenzoyl)oxy-5-chloro-4-pyridyl, 2-(4-fluorobenzoyl)oxy-5-chloro-4-pyridyl, 2-(2,4-dichlorobenzoyl)oxy-5-chloro-4-pyridyl, 2-(3,4,5-trichlorobenzoyl)oxy-5-chloro-4-pyridyl, 2-(2-methoxybenzoyl)oxy-5-chloro-4-pyridyl, 2-(3-methoxybenzoyl)oxy-5-chloro-4-pyridyl, 2-(4-methoxybenzoyl)oxy-5-chloro-4-pyridyl, 2-(3,4,5-trimethoxybenzoyl)oxy-5-chloro-4-pyridyl, 4-benzoyloxy-5-chloro-2-pyridyl, 4-benzoyloxy-5-fluoro-2-pyridyl, 4-benzoyloxy-5-bromo-2-pyridyl, 4-benzoyloxy-5-iodo-2-pyridyl, 4-benzoyloxy-5-methyl-2-pyridyl, 4-benzoyloxy-5-cyano-2-pyridyl, 4-benzoyloxy-5-nitro-2-pyridyl, 4-benzoyloxy-5-carboxy-2-pyridyl, 3-benzoyloxy-5-chloro-2-pyridyl, 6-benzoyloxy-5-chloro-2-pyridyl, 6-(2,4-dichlorobenzoyloxy)-3-cyano-2-pyridyl, 6-(3,4,5-trimethoxybenzoyloxy)-3-cyano-2-pyridyl, 6-(4-fluorobenzoyloxy)-3-chloro-2-pyridyl, 6-benzoyloxy-3-cyano-2-pyridyl, 4-(4-methylbenzoyl)oxy-5-chloro-2-pyridyl, 4-(2,4-dimethylbenzoyl)oxy-5-chloro-2-pyridyl, 4-(3,4,5-trimethylbenzoyl)oxy-5-chloro-2-pyridyl, 4-(2-chlorobenzoyl)oxy-5-chloro-2-pyridyl, 4-(2,4-dichlorobenzoyl)oxy-5-chloro-2-pyridyl, 4-(4-methoxybenzoyl)oxy-5-chloro-2-pyridyl, 1-phthalidyl-1,2-dihydro-2-oxo-3-pyridyl, 1-phthalidyl-1,2-dihydro-2-oxo-4-pyridyl, 1-phthalidyl-1,2-dihydro-2-oxo-5-pyridyl, 1-phthalidyl-1,2-dihydro-2-oxo-6-pyridyl, 1-carbomethoxymethylcarbamoyl-1,2-dihydro-2-oxo-3-pyridyl, 1-carbomethoxymethylcarbamoyl-1,2-dihydro-2-oxo-4-pyridyl, 1-carbomethoxymethylcarbamoyl-1,2-dihydro-2-oxo-5-pyridyl, 1-carbomethoxymethylcarbamoyl-1,2-dihydro-2-oxo-6-pyridyl, 1-carboethoxymethylcarbamoyl-1,2-dihydro-2-oxo-4-pyridyl, 2-hydroxy-5-methyl-3-pyridyl, 2-hydroxy-5-methyl-4-pyridyl, 2-hydroxy-5-methyl-6-pyridyl, 2-hydroxy-5-ethyl-4-pyridyl, 2-hydroxy-3-methyl-4-pyridyl, 2-hydroxy-3-cyano-4-pyridyl, 2-hydroxy-3-cyano-5-pyridyl, 2-hydroxy-3-cyano-6-pyridyl, 2-hydroxy-5-cyano-6-pyridyl, 2-hydroxy-3-nitro-4-pyridyl, 2-hydroxy-3-nitro-5-pyridyl, 2-hydroxy-5-nitro-6-pyridyl, 2-hydroxy-6-nitro-4-pyridyl, 2-hydroxy-5-carboxy-3-pyridyl, 2-hydroxy-5-carboxy-4-pyridyl, 2-hydroxy-5-carboxy-6-pyridyl, 2-hydroxy-3-carboxy-4-pyridyl, 2-hydroxy-5-ethoxycarbonyl-3-pyridyl, 2-hydroxy-5-ethoxycarbonyl-4-pyridyl, 2-hydroxy-5-ethoxycarbonyl-6-pyridyl, 2-hydroxy-3-ethoxycarbonyl-4-pyridyl, 2-hydroxy-3,5-dichloro-4-pyridyl, 2-hydroxy-3,5-dichloro-6-pyridyl, 2-hydroxy-3,5-dibromo-4-pyridyl, 2-hydroxy-3,5-dibromo-6-pyridyl, 2-hydroxy-3-amino-4-pyridyl, 2-hydroxy-3-amino-5-pyridyl, 2-hydroxy-3-amino-6-pyridyl, 2-hydroxy-3-carbamoyl-4-pyridyl, 2-hydroxy-3-carbamoyl-5-pyridyl, 2-hydroxy-3-carbamoyl-6-pyridyl, 2,4-dihydroxy-6-pyridyl, 2,6-dihydroxy-4-pyridyl, 1,2-dihydro-2-oxo-4-pyridyl, 1,6-dihydro-6-oxo-2-pyridyl, 1-(benzyloxymethyl)-5-chloro-1,2-dihydro-2-oxo-4-pyridyl, 1-(benzyloxymethyl)-5-fluoro-1,2-dihydro-2-oxo-6-pyridyl, 1-(β-phenethyloxymethyl)-5-bromo-1,2-dihydro-2-oxo-4-pyridyl, 1-(2-benzyloxyethyl)-5-chloro-1,2-dihydro-2-oxo-4-pyridyl, 5-chloro-4-[p-(N,N-dimethylamino)benzoyloxy]-2-pyridyl, 5-bromo-4-[p-(N,N-dimethylamino)benzoyloxy]-2-pyridyl, 5-chloro-4-[p-(N,N-dimethylamino)benzoyloxy]-3-pyridyl, 5-chloro-4-[p-(N-methyl-N-ethylamino)benzoyloxy]-2-pyridyl, 5-fluoro-4-[o-(N,N-dimethylamino)benzoyloxy]-2-pyridyl, 5-fluoro-4-hexanoyloxy-2-pyridyl, 5-bromo-4-hexanoyloxy-2-pyridyl, 5-iodo-4-hexanoyloxy-2-pyridyl, 5-fluoro-4-octadecanoyloxy-2-pyridyl, 5-chloro-4-octadecanoyloxy-2-pyridyl, 5-bromo-4-octadecanoyloxy-2-pyridyl, 5-iodo-4-octadecanoyloxy-2-pyridyl, 5-fluoro-4-(2-furoyloxy)-2-pyridyl, 5-chloro-4-(2-furoyloxy)-2-pyridyl, 5-bromo-4-(2-furoyloxy)-2-pyridyl, 5-iodo-4-(2-furoyloxy)-2-pyridyl, 5-chloro-4-(3-furoyloxy)-2-pyridyl, 5-bromo-4-(3-furoyloxy)-2-pyridyl, 5-fluoro-4-(2-thenoyloxy)-2-pyridyl, 5-chloro-4-(2-thenoyloxy)-2-pyridyl, 5-bromo-4-(2-thenoyloxy)-2-pyridyl, 5-iodo-4-(2-thenoyloxy)-2-pyridyl, 5-chloro-4-(3-thenoyloxy)-2-pyridyl, 5-iodo-4-(3-thenoyloxy)-2-pyridyl, 4-(1-naphthoyloxy)-2-pyridyl, 4-(2-naphthoyloxy)-2-pyridyl, 5-(1-naphthoyloxy)-2-pyridyl, 5-(2-naphthoyloxy)-2-pyridyl, 6-(2-naphthoyloxy)-2-pyridyl, 3-cyano-6-(2-thenoyloxy)-2-pyridyl, 3-cyano-6-(3-thenoyloxy)-2-pyridyl, 4-cyano-6-(2-thenoyloxy)-2-pyridyl, 3-cyano-6-(2-furoyloxy)-2-pyridyl and the like.

Arylene groups represented by Y in the pyridyloxycarbonyl-arylene carbonyl group of the formula (Y) include those formed from aromatic hydrocarbons or 5- or 6- membered aromatic heterocycles containing one or two heteroatoms which are the same or different and which is or are selected from the group consisting of nitrogen and oxygen by removing two hydrogen atoms each bonded to the two different carbon atoms. Examples of such arylene groups are phenylene groups such as 1,2-phenylene, 1,3-phenylene, 1,4-phenylene and the like; naphthylene groups such as 1,2-naphthylene, 1,3-naphthylene, 1,4-naphthylene, 1,5-naphthylene, 1,6-naphthylene, 1,7-naphthylene, 1,8-naphthylene and the like; pyridinediyl groups such as 2,4-pyridinediyl, 2,5-pyridinediyl, 2,6-pyridinediyl, 3,4-pyridinediyl, 3,5-pyridinediyl and the like; pyrazinediyl groups such as 2,3-pyrazinediyl, 2,6-pyrazinediyl, 2,5-pyrazinediyl, and the like; furandiyl groups such as 2,3-furandiyl, 3,4-furandiyl, 2,5-furandiyl, and the like; 4-pyridon-1-lower alkyl-diyl groups such as 4-pyridon-1-methyl-2,3-diyl, 4-pyridon-1-methyl-2,5-diyl, 4-pyridon-1-methyl-2,6-diyl and the like.

With respect to the formula (1b), the substituent α includes 5-fluorouracil derivative groups so far as it can be converted to 5-fluorouracil in vivo and so far as it can form an ester or amide linkage when taken together with the carbonyl group to which this substituent α is attached; i.e., suitable 5-fluorouracil derivative groups include those formed from the 5-fluorouracil derivatives represented by the formulas (1-1a) and (1-1c).
(i) A compound of the formula 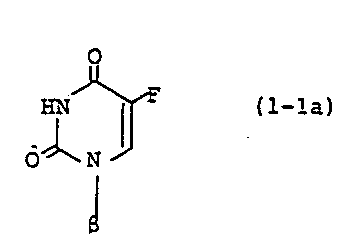 wherein β represents hydrogen atom, tetrahydrofuranyl, lower alkylcarbamoyl, phthalidyl, lower alkoxy-lower alkyl or lower alkanoyloxy-lower alkoxycarbonyl group.
   The compound of the formula (1-1a) is disclosed in J. Med. Chem. 25, 1219, 1982, Cancer Chemother. Pharmacol., 1, 203-208, 1978 and Japanese Unexamined Patent Publication No. 37787/1975.
   With respect to the formula (1-1a), 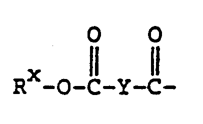 group can be linked by an amide linkage to the 3- or 1-position of the 5-fluorouracil ring.
(ii) A compound of the formula 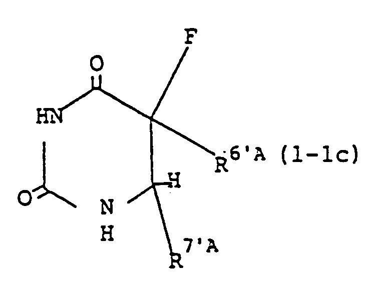 wherein R^{6'A} represents lower alkoxycarbonyl group, R^{7'A} represents lower alkoxy group or group

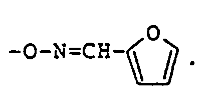

The compound of the formula (1-1c) is disclosed in EPC application published under the publication No. 99091.

With respect to the formula (1-1c), 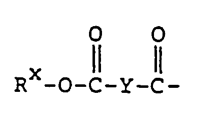
group can be linked by an amide linkage to the 1- or 3-position of the 5-fluorouracil ring.

With respect to the compounds of the formula (1-1a) and (1-1c), the groups represented by β, R^{6'A} and R^{7'A} are exemplified below.

Examples of lower alkylcarbamoyl groups are alkylcarbamoyl groups having one or two C₁-C₆ alkyl groups such as N-methylcarbamoyl, N-ethylcarbamoyl, N-propylcarbamoyl, N-isopropylcarbamoyl, N-butylcarbamoyl, N-tert-butylcarbamoyl, N-pentylcarbamoyl, N-hexylcarbamoyl, N,N-dimethylcarbamoyl, N,N-diethylcarbamoyl, N,N-dipropylcarbamoyl, N-isopropyl-N-methylcarbamoyl, N-ethyl-N-methylcarbamoyl, N-methyl-N-pentyl-carbamoyl, N-propyl-N-pentylcarbamoyl, N,N-dipentylcarbamoyl, N-ethyl-N-hexylcarbamoyl, N-hexyl-N-pentylcarbamoyl, N,N-dihexylcarbamoyl and the like.

Examples of lower alkoxy-lower alkyl groups are alkoxyalkyl groups in which the alkoxy moiety and alkyl moiety each have 1 to 6 carbon atoms, such as methoxymethyl, 3-methoxypropyl, 4-ethoxybutyl, 6-propoxyhexyl, 5-isopropoxypentyl, 1,1-dimethyl-2-butoxyethyl, 2-methyl-3-tert-butoxypropyl, 2-pentyloxyethyl, 2-hexyloxyethyl and the like.

Examples of lower alkanoyloxy-lower alkoxycarbonyl groups are alkanoyloxy-alkoxycarbonyl groups in which the alkanoyl moiety and alkoxy moiety each have 1 to 6 carbon atoms, such as acetyloxymethoxycarbonyl, 4-(formyloxy)butoxycarbonyl, 6-(propionyloxy)hexyloxycarbonyl, 5-(isobutyryloxy)pentyloxycarbonyl, 6-(hexanoyloxy)hexyloxycarbonyl, 2-(hexanoyloxy)ethoxycarbonyl, 2-(acetyloxy)ethoxycarbonyl, acetyloxymethoxycarbonyl and the like.

Examples of lower alkoxycarbonyl groups are carbonyl groups having C₁-C₆ alkoxy group such as methoxycarbonyl, ethoxycarbonyl, propoxycarbonyl, isopropoxycarbonyl, tert-butoxycarbonyl, butoxycarbonyl, pentyloxycarbonyl, hexyloxycarbonyl and the like.

The compounds represented by the formula 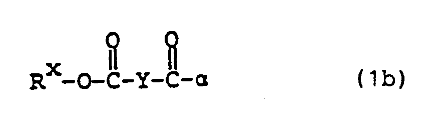
wherein R^{x}, Y and α are as defined above, can be prepared by the processes represented by the following reaction schemes (a) and (b).

Reaction scheme (a) 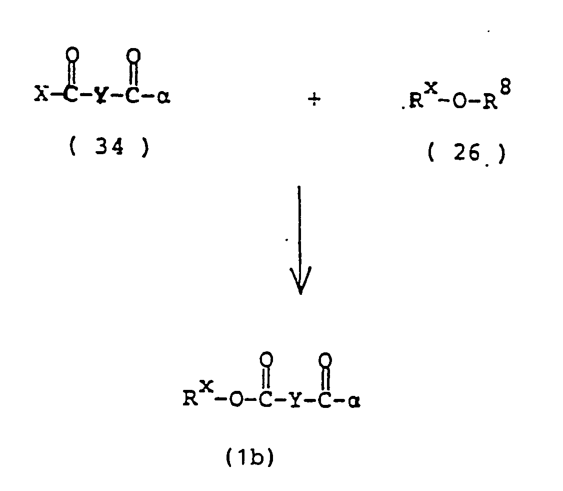
wherein X is a halogen atom, R⁸ is hydrogen or tri(lower alkyl) silyl group and α, R^{x} and Y are as defined above.

First acid halides (34a) and (34c) are prepared by introducing an acyl group XCO-Y-CO- into 3-position of the compounds of the formulae (1-1a) and (1-1c) in accordance with the following reaction schemes (i₁) and (i₂).

Reaction scheme (i₁): 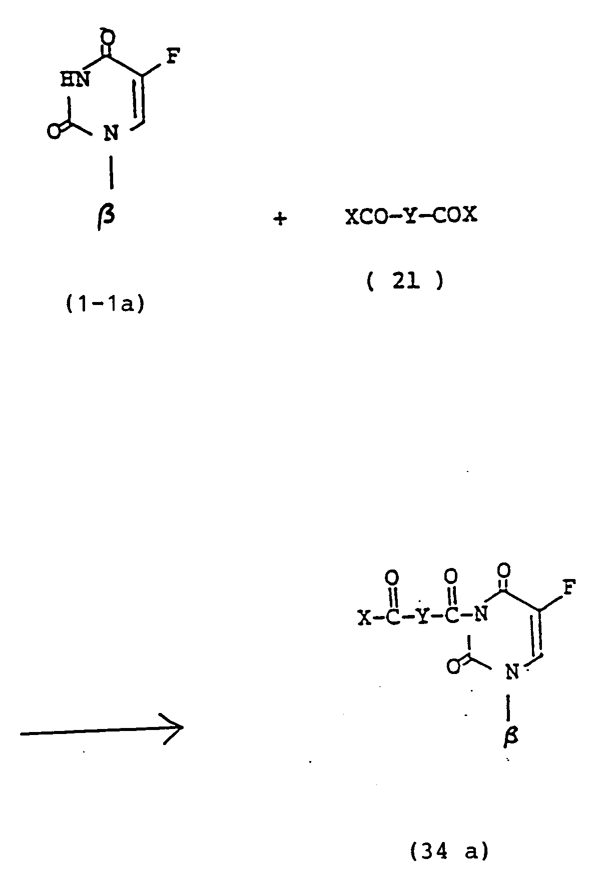
wherein X is a halogen atom, and β and Y are as defined above.

Reaction scheme (i₂): 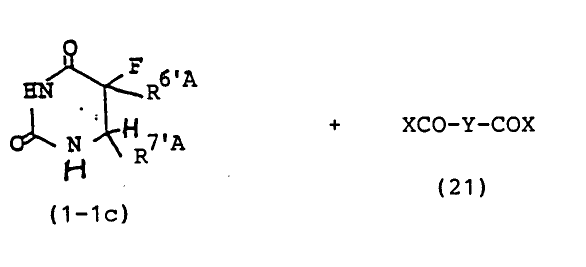 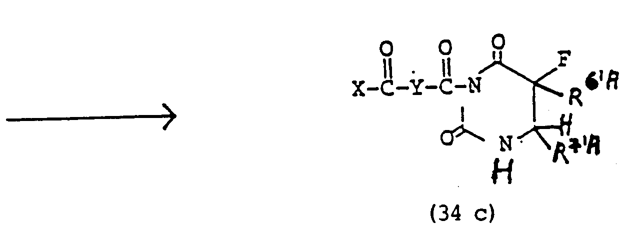
wherein X is a halogen atom, and R^{6'A}, R^{7'A} and Y are as defined above.

The present reaction, wherein acyl (XCO-Y-CO-) is introduced into the 3-position of the pyrimidine moiety (acylation), can be conducted by a usual process, e.g., the acid halide process. With the acid halide process, an acyl halide (XCO-Y-COX) is caused to act on the compounds (1-1a) and (1-1c) in a suitable solvent in the presence of an acid scavenger to give the desired compounds 34a and 34c. Examples of useful acid scavengers are sodium hydrogen carbonate, sodium carbonate, potassium carbonate, pyridine, triethylamine, etc. Examples of useful solvents are benzene, chloroform, methylene chloride, carbon tetrachloride, dioxane, tetrahydrofuran, etc. The acyl halide is used in an amount of at least about one mole, preferably about 1 to about 3 moles, per mole of the compounds (1-1a) and (1-1c). The reaction temperature is usually -30 to 100°C, preferably room temperature to about 80°C. The reaction takes about 20 minutes to about 20 hours. Then the resulting compounds (34a) and (34c) are reacted with the compound (26) to give (1b).

When the compound (26) used is one wherein R⁸ is hydrogen, the reaction can be carried out in the same manner as in the preparation of compounds (34a) and (34c).

Further when the compound (26) used is one wherein R⁸ is tri(lower alkyl)silyl group, the reaction is conducted using a solvent, which will not adversely affect the reaction, in a dry state, preferably in an anhydrous state. Examples of preferred solvents are halogenated hydrocarbon such as dichloromethane and chloroform, aromatic hydrocarbons such as benzene and toluene, and aprotic solvents such as dioxane, acetonitrile, acetone and tetrahydrofuran. Usually, at least about one mole, preferably 1 to 3 moles, of the compound (26) is used per mole of the acid halides (34a) and (34c). The reaction is conducted at about 0 to about 100°C, preferably room temperature to about 80°C, for 1 to 6 hours, preferably about 2 hours. When R⁸ is hydrogen, it is desirable to use a base, such as triethylamine, trimethylamine, dimethylaniline or pyridine, for the reaction. Further if R⁸ is tri(lower alkyl)silyl, it is desirable to use a Lewis acid such as stannic chloride, zinc chloride or aluminum chloride.

The compounds (26) to be used for the present reaction are generally those known but include some novel compounds. Such compounds can be easily prepared by the following process. The compound substituted with 2-tetrahydrofuranyl at the 1-position of the pyridine ring can be prepared from a corresponding known pyridine derivative having a hydrogen atom at the 1-position, by adding hexamethyldisilazane to the derivative, refluxing the mixture for about 2 to about 20 hours, thereafter removing the excess of the silazane from the reaction mixture, dissolving the residue in halogenated hydrocarbon such as dichloromethane and chloroform or in an aprotic solvent such as dioxane and acetonitrile adding to the solution 2-acetoxytetrahydrofuran in an amount of at least about one mole to 2 moles per mole of the pyridine derivative and a Lewis acid such as stannic chloride, zinc chloride or aluminum chloride, and reacting the mixture at about 0 to about 100°C, preferably around room temperature for about 1 to about 10 hours. When 2-acetoxytetrahydrofuran used for the reaction is replaced by an aroyl halide, lower alkanoyl halide, aroyloxycarbonyl halide, lower alkoxycarbonyl halide, 2-halogenophthalide allyl halide, phenyl-lower alkoxy-lower alkyl halide or lower alkoxy-lower alkyl halide, the compound obtained has the substituent of aroyloxy group, lower alkanoyloxy group, aroyloxycarbonyloxy group or lower alkoxycarbonyloxy group at the 2 and/or 4 position(s) of the pyridine ring, or of phthalidyl group, allyl group, phenyl-lower alkoxy-lower alkyl group or lower alkoxy-lower alkyl group at the 1-position of the pyridine ring. Further the compound substituted with N-(lower alkoxycarbonyl lower alkyl)aminocarbonyl at the 1-position of the pyridine ring can be prepared from a corresponding pyridine derivative having a hydrogen atom at the 1-position, by adding to the derivative with at least about one mole of a lower alkoxycarbonyl-lower alkyl-isocyanate per mole of the derivative in a solvent such as dioxane, dichloromethane, chloroform, acetonitrile, acetone or pyridine, and subjecting the mixture to reaction at about 0 to about 100°C for about 10 minutes to about 1 hour, preferably about 30 minutes.

Reaction scheme (b) 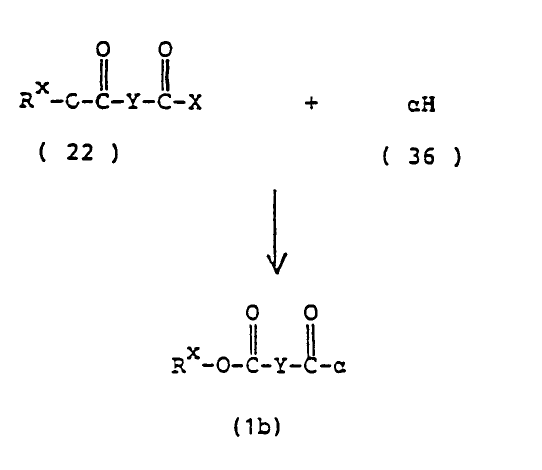
wherein R^{x}, Y and α are as defined above and X is a halogen atom.

This reaction can be carried out in the same manner as the reaction of the schemes (i₁) or (i₂).

The compounds (1b) of the present invention have an outstanding anticancer effect and are low in toxicity. For example, they are reduced in side effects such as loss of body weight. The present compounds are therefore very useful as antitumor agents for curing cancers in man and animals.

Our research has revealed that the anticancer effect of the present compounds (1) can be further enhanced when they are used in combination with a pyridine derivative represented by the formula (X) below. 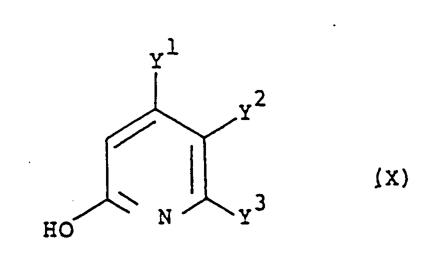
wherein one of Y¹ and Y³ is a hydrogen atom, the other is hydroxyl, Y² is a hydrogen atom, halogen atom, lower alkyl, cyano or nitro, and the hydroxyl group(s) at the 2-, 4- and/or 6-position(s) may be an acylated one.

Some of the compounds of the formula (X) are known. The others can be easily prepared by a known process (Triv. Chem. Rec. 73, 704 (1954)). The acylation of the hydroxyl group(s) at the 2-, 4- and/or 6-position(s) can be conducted in the same manner as in reaction scheme (b) or (c).

The desired products of the present invention are used in the form of generally acceptable pharmaceutical compositions which are prepared by using diluents and excipients such as fillers, bulking agents, binders, wetting agents, disintegrating agents, surfactants, lubricnats and the like. Administration unit forms of these pharmaceutical compositions of the present invention can be varied and selected so as to meet various therapeutical purposes. Typical forms of the pharmaceutical compositions can be exemplified such as tablets, pills, powders, liquids, suspensions, emulsions, granules, capsules, suppositories, injections (liquids, suspensions and others), ointments and the like.

In shaping into the form of tablets, those known as the carriers in this field can widely be applied for example, excipients such as lactose, purified sugar, sodium chloride, glucose, urea, starch, calcium carbonate, kaolin, crystalline cellulose, silicic acid and others; binders such as water, ethanol, propanol, simple syrup, a glucose solution, starch solution, gelatin solution, carboxymethyl cellulose, shellac, methyl cellulose, potassium phosphate, polyvinylpyrrolidone and others; disintegrating agents such as dried starch, sodium alginate, agar-agar powder, laminaran powder, sodium hydrogen carbonate, calcium carbonate, a fatty acid ester of polyoxyethylene sorbitan, sodium laurylsulfate, monoglyceride of stearic acid, starch, lactose and others; disintegration inhibitors such as purified sugar, stearing cacao butter, hydrogenated oils and others; absorption accelerators such as quaternary ammonium base, sodium laurylsulfate and others; wetting agents such as glycerin, starch and others; adsorption accelerators such as starch, lactose, kaolin, bentonite, colloidal silicic acid and others; and lubricants such as purified talc powder, stearic acid salts, boric acid powder, polyethylene glycol and others can be exemplified. If necessary, the tablets can further be coated with usual coating film to make them into coated tablets, for example sugar-coated tablets, gelatin film-coated tablets, enteric film-coated tablets, film-coated tablets, or double-layered tablets, multiple-layered tablets and others. In shaping into the form of pills, those known as the carriers in this field can widely be applied for example, excipients such as glucose, lactose, starch, cacao butter, hydrogenated vegetable oils, kaolin, talc and others; binders such as powdered gum arabi, powdered tragacanth gum, gelatin, ethanol and others; disintegrating agent such as laminaran, agar-agar powder and others. In shaping into the form of suppositories, those known in this field can widely be applied for example, polyethylene glycol, cacao butter, a higher alcohol, an ester of a higher alcohol, gelatin, semi-synthesized glyceride and others. Capsules are prepared in a conventional manner by admixing the compound of the invention with the foregoing various carriers and encapsulating the mixture into hard-gelatin capsules, soft-gelatin capsules, etc. In case of preparing injections, solutions, emulsions and suspensions being prepared are sterilized, and they are preferably isotonic to the blood. In preparing into the form of solutions emulsions and suspensions, those known as the diluents in this field car widely be applied, for example water, ethanol, propylene glycol, ethoxylated isostearyl alcohol, polyoxylated isostearyl alcohol, a polyoxyethylene sorbitan fatty acid ester and others. In case of preparing isotonic solutions, sufficient amount of sodium chloride, glucose or glycerin may be added to make the solution to be isotonic to the blood. The pharmaceutical compositions for injection preparation may further be contain usual dissolving agents, buffer solutions, analgesic agents or the like if necessary. The pharmaceutical composition of the present invention may also contain coloring agents, preservatives, perfumes, seasoning agents, sweetening agents and others, as well as contain other medicines, if necessary. In shaping into the form of pastes, creams and gels, diluents such as white vaseline, paraffins, glycerine, cellulose derivatives, polyethylene glycols, silicones, bentonite and the like can be used.

The amount of the desired product according to the present invention to be contained as the active ingredient in the pharmaceutical composition is not specifically restricted and can be selected from a wide range, generally 1 to 70 % by weight, may be used.

Administration method of the above-mentioned pharmaceutical composition is not specifically restricted; it can be administered through a suitable method for the respective types of administration forms, depending upon age of the patient, distinction of the sex and other conditions, conditions of the patient and others. For example, tablets, pills, liquids, suspensions, emulsions, granules and capsules are administered orally; injections are administered intraveneously singly or as a mixture with usual injectable transfusions such as a glucose solution, amino acid solutions, and others; and if necessary the injections are administered singly intramuscularly, intracutaneously, subcutaneously or intraperitoneally; and the suppositories are administered into rectum.

The dosage of the desired products of the present invention may suitably be selected depending upon the method for administration, age of the patient, distinction of sex and other conditions, and conditions of the symptoms, and generally the pharmaceutical compositions of the invention can be administered in an amount of about 0.5 to about 20 mg/kg of the body weight/day, calculated as the compound of the invention (active ingredient), in 1 to 4 divided doses.

The present invention will be described in greater detail with reference to the following reference examples, examples, pharmaceutical tests and preparation examples.

In connection with the NMR data in the reference examples and examples, the numerals used as a subscript at the right of the symbol "C", "H" or "N" are used to refer to the position in the compound. Thus the term "C₆-H", for example, refers to the hydrogen bonded to the carbon atom at the 6-position. Similarly the term "C_{3'·4'·5'}-H", for example, denotes the hydrogens bonded to the carbon atoms at the 3'-, 4'- and 5'-positions. Also the term "H₁", for example, refers to the hydrogen bonded to the carbon atom at the 1-position. The term "N₃-H", for example, refers to the hydrogen bonded to the nitrogen atom at the 3-position of the 5-fluorouracil ring.

### Reference Example 1

### Preparation of 5-chloro-4-hydroxy-1-(2-tetrahydrofuranyl)-2(1H)-pyridone

A 10 ml quantity of hexamethyldisilazane was added to 1.00 g of 5-chloro-4-hydroxy-2(1H)-pyridone, and the mixture was refluxed for 6 hours. Then excess hexamethyldisilazane was distilled off and the oily residue was dissolved in 50 ml of dichloromethane. To the solution was added 1.00 g of 2-acetoxytetrahydrofuran and 0.1 ml of stannic chloride and the mixture was stirred overnight at room temperature. The reaction mixture was neutralized with triethylamine, and the solvent was distilled off. Methanol was added to the residue and the mixture was stirred at room temperature for 2 hours. The solvent was distilled off and the residue was placed on a silica gel column and eluted with 2 % methanol-chloroform, giving 1.07 g of the title compound in a yield of 73.5 %.
M.p. 170-173°C
¹H-NMR(DMSO-d₆)δ:
11.6 (1H, bs, OH)
7.59 (1H, s, C₆-H)
6.09-5.99 (1H, q, 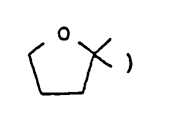
5.76 (1H, s, C₃-H)
4.39-3.73 (2H, m, 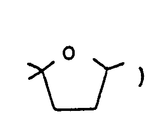
2.42-1.82 (4H, m,

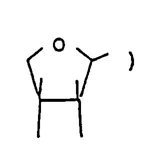

### Reference Example 2

### Preparation of 5-chloro-1-ethoxymethyl-4-hydroxy-2(1H)-pyridone

Following the general procedure of Reference Example 3 and using chloromethyl ethyl ether in place of the 2-acetoxytetrahydrofuran, 5-chloro-1-ethoxymethyl-4-hydroxy-2(1H)-pyridone was prepared in a yield of 39 %.
M.p. 217-219°C
¹H-NMR (DMSO-d₆)δ:
11.63 (1H, bs, OH)
7.87 (1H, s, C₆-H)
5.75 (1H, s, C₃-H)
5.16 (2H, s, N-CH₂-O-)
3.49 (2H, q, J = 7Hz, -OCH₂ CH₃)
1.09 (3H, t, J = 7Hz, -OCH₂ CH₃)

### Reference Example 3

### Preparation of 2-benzoyloxy-5-chloro-4-hydroxypyridine

The title compound was prepared in a yield of 51 % by following the general procedure of Reference Example 1 and using benzoyl chloride in place of the 2-acetoxytetrahydrofuran. The title compound softens at 184°C.
¹H-NMR (DMSO-d₆)δ:
8.27 (1H, s, C₆-H)
8.16-8.07 (2H, m, 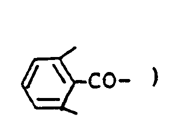
7.78-7.51 (3H, m, 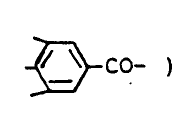
6.91 (1H, s, C₃-H)

### Reference Example 4

### Preparation of 4-hydroxy-1-(3-phthalidyl)-2(1H)-pyridone

A 10 ml quantity of hexamethyldisilazane was added to 1.00 g of 4-hydroxy-2(1H)-pyridone and the mixture was refluxed for 6 hours. Then the reaction mixture was concentrated under reduced pressure and the concentrate was dissolved in 20 ml of acetonitrile. To this solution was added 2.29 g of 3-bromophthalide and the mixture was stirred overnight at room temperature. Methanol was added to the reaction mixture and the resulting mixture was stirred at room temperature for 15 minutes. Then the reaction mixture was concentrated under reduced pressure, and the concentrate was applied to a silica gel column and eluted with 1 % methanol-chloroform, giving 0.62 g of the title compound in a yield of 30 %.
M.p. 239-241°C
¹H-NMR(DMSO-d₆)δ:
11.05 (1H, bs, OH)
8.29-7.52 (5H, m, 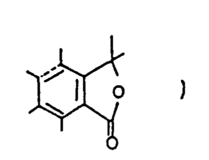
6.99 (1H, d, J = 8Hz, C₆-H)
5.88 (1H, dd, J₃·₅ = 2Hz, J₅·₆ = 8Hz, C₅-H)
5.65 (1H, d, J = 2Hz, C₃-H)

### Reference Example 5

### Preparation of 1-carbomethoxymethylcarbamoyl-4-hydroxy-2(1H)-pyridone

A 2.49 g quantity of carbomethoxymethylisocyanate was added to a suspension of 2.00 g of 4-hydroxy-2(1H)-pyridone in 60 ml of dioxane, and the mixture was stirred at 90°C for 30 minutes. The solvent was distilled off, and ether was added to the residue. The precipitate thus formed was filtered, giving 2.20 g of the title compound in a yield of 54 %.
M.p. 124 - 126°C
¹H-NMR(DMSO-d₆)δ:
11.52 (1H, bs, OH)
10.82 (1H, t, J = 6Hz, -CONHCH₂-)
8.20 (1H, d, J = 8Hz, C₆-H)
6.17 (1H, dd, J₅·₆ = 8Hz, J₃·₅ = 2Hz, C₅-H)
5.74 (1H, d, J = 2Hz, C₃-H)
4.15 (2H, d, J = 6Hz, -CONHCH₂-)
3.68 (3H, s, -COOCH₃)

### Reference Example 6

### Preparation of 1-benzyloxymethyl-5-chloro-4-hydroxy-2(1H)-pyridone

Following the general procedure of Reference Example 3 and using benzyloxymethyl chloride in place of 2-acetoxytetrahydrofuran and also using acetonitrile as the solvent in place of dichloromethane, the title compound was prepared in a yield of 57 %.
M.p. 165-167°C
¹H-NMR(DMSO-d₆)δ:
11.65 (1H, bs, OH)
7.92 (1H, s, C₆-H)
7.31 (5H, s, phenyl-H)
5.77 (1H, s, C₃-H)
5.27 (2H, s, -NCH₂O-)
4.55 (2H, s,

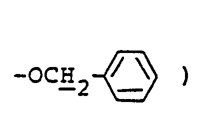

### Reference Example 7

### Preparation of 2,4-bis(trimethylsilyloxy)-5-chloropyridine

A 50 ml quantity of hexamethyldisilazane was added to 9.6 g of 5-chloro-4-hydroxy-2(1H)-pyridone, and the mixture was stirred overnight on an oil bath at 140°C. The insolubles were removed by filtration, and the filtrate was distilled under reduced pressure, giving 14.4 g of the title compound boiling at 120°C/7 mmHg.
Yield 75 %.

### Reference Example 8

### Preparation of 2-acetoxy-5-chloro-4-hydroxypiridine

A 2.09 ml quantity of acetyl bromide and 0.10 ml of stannic chloride were added to a solution of 5.00 g of 2,4-bis(trimethylsilyloxy)-5-chloropyridine in 250 ml of dry dichloromethane, and the mixture was stirred at room temperature for 3.5 hours. The reaction mixture was neutralized with triethylamine, and the solvent was distilled off. The residue was applied to a silica gel column and eluted with 40 % ethyl acetate-benzene as an eluent, giving 2.07 g of the title compound in a yield of 64 %.
M.p. 270-272°C
¹H-NMR(DMSO-d₆)δ:
11.90 (1H, bs, OH)
8.20 (1H, s, C₆-H)
6.69 (1H, s, C₃-H)
2.27 (3H, s, COCH₃)

### Example 1

### Preparation of 3-[3-(1,2-dihydro-2-oxo-4-pyridyloxycarbonyl)benzoyl]-5-fluoro-1-(2-tetrahydrofuranyl)uracil

A 1.00 g quantity of isophthaloyl chloride and 0.70 ml of triethylamine were added to a solution of 1.00 g of 5-fluoro-1-(2-tetrahydrofuranyl)uracil in 30 ml of dried dioxane. The mixture was refluxed for 2 hours. Thereto added was 1.00 ml of triethylamine and the mixture was refluxed for 2 hours. Thereto further added was 0.60 g of 4-hydroxy-2(1H)-pyridone and the mixture was refluxed for 3 hours. The solvent was distilled off and 30 ml of ethyl acetate and 30 ml of water were added to the residue. The insolubles were removed by filtration. The ethyl acetate layer was separated, dried over anhydrous sodium sulfate and concentrated. The concentrate was subjected to silica gel column chromatography to conduct gradient elution using chloroform and mixtures of methanol (up to 2 %) and chloroform. The fractions corresponding to the title compound were collected and concentrated, giving 0.40 g of a powder in a yield of 18 %.
¹H-NMR(DMSO-d₆)δ:
11.74 (1H, bs, -NH-, disappeared by addition of D₂O)
8.63-8.41 (3H, m, C₂·₄·₆-H of the benzoyl ring)
8.14 (1H, d, J = 7Hz, C₆-H)
7.84 (1H, t, J = 8Hz, C₃-H of the benzoyl ring)
7.51 (1H, d, J = 8Hz, C₆-H of the pyridine ring)
6.34-6.26 (2H, m, C₃·₅-H of the pyridine ring)
5.93 (1H, t, J = 4Hz, C_{1'}-H)
4.41-4.20 and 3.92-3.72 (each 1H, m, C_{4'}-H)
2.27-1.92 (4H, m, C_{2'}·_{3'}-H)

### Example 2

### Preparation of 3-[4-(1,2-dihydro-2-oxo-4-pyridyloxycarbonyl)benzoyl]-5-fluoro-1-(2-tetrahydrofuranyl)uracil

The title compound was prepared in the same manner as in Example 1.
¹H-NMR(DMSO-d₆)δ:
11.73 (1H, bs, -NH-, disappeared by addition of D₂O)
8.28 (4H, s, phenyl-H)
8.14 (1H, d, J = 7Hz, C₆-H)
7.52 (1H, d, J = 8Hz, C₆-H of the pyridine ring)
6.33-6.25 (2H, m, C₃·₅-H of the pyridine ring)
5.92 (1H, bt, J = 4Hz, C_{1'}-H)
4.49-4.26 and 4.03-3.77 (each 1H, m, C_{4'}-H)

### Example 3

### Preparation of 3-[3-(2-benzoyloxy-5-chloro-4-pyridyloxycarbonyl)benzoyl]-5-fluoro-1-(2-tetrahydrofuranyl)uracil

The title compound was prepared in the same manner as in Example 1.
¹H-NMR(CDCl₃)δ:
8.69-8.16 (6H, m, 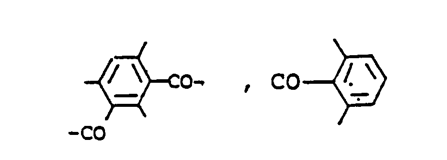
and C₆-H of the pyridine ring)
7.81-7.45 (5H, m, 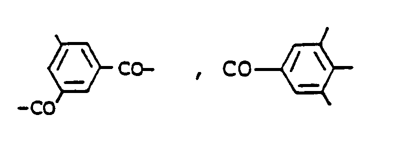
and C₆-H)
7.41 (1H, s, C₃-H of the pyridine ring)
6.00-5.90 (1H, m, C_{1'}-H)
4.42-3.87 (2H, m, C_{4'}-H)
2.55-1.89 (4H, m, C_{2'·3'}-H)

### Example 4

### Preparation of 3-[3-(4-benzoyloxy-5-chloro-2-pyridyloxycarbonyl)benzoyl]-5-fluoro-1-(2-tetrahydrofuranyl)uracil

The title compound was prepared in the same manner as in Example 1.
¹H-NMR(CDCl₃)δ:
8.66-8.17 (6H, m, 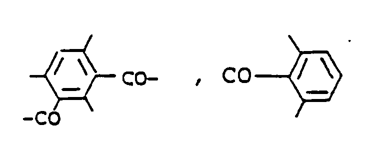
and C₆-H of the pyridine ring)
7.79-7.41 (5H, m, 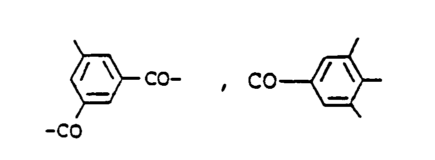
and C₆-H)
7.38 (1H, s, C₃-H of the pyridine ring)
6.02-5.90 (1H, m, C_{1'}-H)
4.45-3.87 (2H, m, C_{4'}-H)
2.55-1.89 (4H, m, C_{2'·3'}-H)

### Example 5

### Preparation of 3-[3-(2-acetoxy-5-chloro-4-pyridyloxycarbonyl)benzoyl]-5-fluoro-1-(2-tetrahydrofuranyl)uracil

A 5.53 ml quantity of triethylamine and 1.52 g of isophthaloyl chloride were added to a solution of 1.00 g of 1-(2-tetrahydrofuranyl)-5-fluorouracil in 50 ml of dried dioxane. The mixture was refluxed for 1 hour. The reaction mixture was filtered and the filtrate was concentrated. The concentrate was dissolved in 50 ml of acetonitrile. To the solution were added 3.46 ml of triethylamine and 1.40 g of 2-acetoxy-5-chloro-4-hydroxypyridine. The mixture was stirred at room temperature for 2 hours. The reaction mixture was filtered and concentrated. The concentrate was purified by silica gel column chromatography using chloroform as an eluent, giving 530 mg of the title compound in a yield of 20 %.
¹H-NMR(CDCl₃)δ:
8.68-8.22 (3H, m, 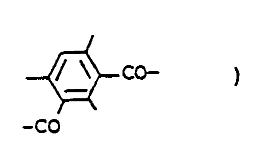
8.47 (1H, s, C₆-H of the pyridine ring)
7.72 (1H, t, J = 8Hz, 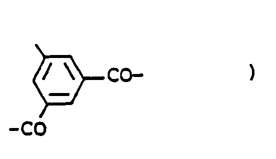
7.54 (1H, d, J = 6Hz, C₆-H)
7.27 (1H, s, C₃-H of the pyridine ring)
5.98-5.90 (1H, m, C_{1'}-H)
4.29-4.04 (2H, m, C_{4'}-H)
2.35 (3H, s, COCH₃)
2.43-1.89 (4H, m, C_{2'}·_{3'}-H)

### Example 6

### Preparation of 3-[3-(4-acetoxy-5-chloro-2-pyridyloxycarbonyl)benzoyl]-5-fluoro-1-(2-tetrahydrofuranyl)uracil

The title compound was prepared in the same manner as in Example 5.
¹H-NMR(CDCl₃)δ:
8.65-8.19 (4H, m, 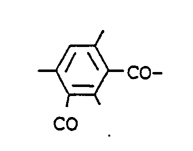
and C₆-H of the pyridine ring)
7.70(1H, t, J = 8Hz, 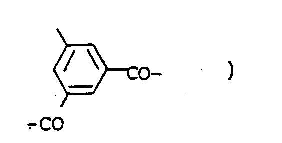
7.53 (1H, d, J = 6Hz, C₆-H)
7.18 (1H, s, C₃-H of the pyridine ring)
6.02-5.90 (1H, m, C_{1'}-H)
4.45-3.87 (2H, m, C_{4'}-H)
2.44-1.90 (7H, m, COCH₃ and C_{2'}·_{3'}-H)

### Example 7

### Preparation of 5-fluoro-3-[3-[2-(2-methylbenzoyloxy)-4-pyridyloxycarbonyl]benzoyl]-1-(2-tetrahydrofuranyl)uracil

The title compound was prepared in the same manner as in Example 5.
¹H-NMR(CDCl₃)δ:
8.67-7.21 (12H, m, phenyl-H, H of the pyridine ring and C₆-H)
6.00-5.90 (1H, m, C_{1'}-H)
4.40-3.90 (2H, m, C_{4'}-H)
2.69 (3H, s, CH₃)
2.60-1.95 (4H, m, C_{2'}·_{3'}-H)

### Example 8

### Preparation of 3-[3-(2-benzoyloxy-4-pyridyloxycarbonyl)benzoyl]-5-fluoro-1-(2-tetrahydrofuranyl)uracil

The title compound was prepared in the same manner as in Example 5.
¹H-NMR(CDCl₃)δ:
8.67-8.18 (6H, m, 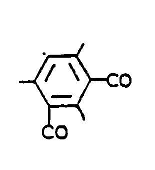
and 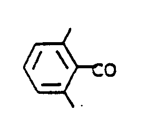
and C₆-H of the pyridine ring)
7.79-7.40 (5H, m, 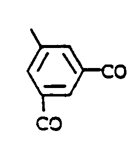
and 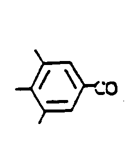
and C₆-H)
7.34-7.23 (2H, m, C₃·₅-H of the pyridine ring)
6.00-5.90 (1H, m, C_{1'}-H)
4.45-3.87 (2H, m, C_{4'}-H)
2.55-1.89 (4H, m, C_{2'·3'}-H)

### Example 9

### Preparation of 3-[3-(1-benzyloxymethyl-5-chloro-1,2-dihydro-2-oxo-4-pyridyloxycarbonyl)benzoyl]-5-fluoro-1-(2-tetrahydrofuranyl)uracil

The title compound was prepared in the same manner as in Example 5.
¹H-NMR(CDCl₃)δ:
8.65-8.21 (3H, m, 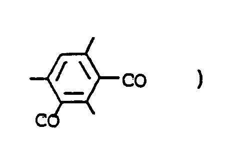
7.79-7.51 (3H, m, 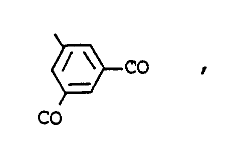
C₆-H, and C₆-H of the pyridine ring)
7.34 (5H, s, -CH₂)
6.65 (1H, s, C₃-H of the pyridine ring)
5.98-5.91 (1H, m, C_{1'}-H)
5.42 (2H, s, NCH₂O-)
4.66 (2H, s, 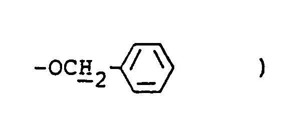
4.27-3.89 (2H, m, C_{4'}-H)
2.57-1.90 (4H, m, C_{2'·3'}-H)

### Example 10

### Preparation of 3-[3-[5-chloro-1,2-dihydro-2-oxo-1-(2-tetrahydrofuranyl)-4-pyridyloxycarbonyl]benzoyl]-5-fluoro-1-(2-tetrahydrofuranyl)uracil

The title compound was prepared in the same manner as in Example 5.
¹H-NMR(CDCl₃)δ:
8.92-8.20 (3H, m, 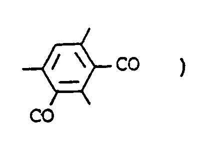
7.79-7.54 (3H, m, 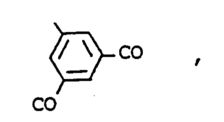
C₆-H and C₆-H of the pyridine ring)
6.58 (1H, s, C₃-H of the pyridine ring)
6.18-5.91 (2H, m, C_{1'}-H of the furanyl group x 2)
4.33-3.87 (4H, m, C_{4'}-H of the furanyl group x 2)
2.67-1.78 (8H, m, C_{2'·3'}-H of the furanyl group x 2)

### Example 11

### Preparation of 5-fluoro-3-[3-[4-(2-naphthoyloxy)-2-pyridyloxycarbonyl]benzoyl]-1-(2-tetrahydrofuranyl)uracil

The title compound was prepared in the same manner as in Example 5.
¹H-NMR(CDCl₃)δ:
8.75-7.20 (15H, m, naphthyl-H, phenyl-H, H of the pyridine ring and C₆-H)
5.97-5.90 (1H, m, C_{1'}-H)
4.52-3.85 (2H, m, C_{4'}-H)
2.52-1.78 (4H, m, C_{2'·3'}-H)

### Example 12

### Preparation of 3-[3-[3-chloro-6-(4-fluorobenzoyloxy)-2-pyridyloxycarbonyl]benzoyl]-5-fluoro-1-(2-tetrahydrofuranyl)uracil

The title compound was prepared in the same manner as in Example 5.
¹H-NMR(CDCl₃)δ:
8.67-8.13 (5H, m, 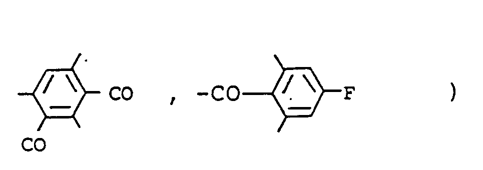
8.00 (1H, d, J = 8Hz, C₄-H of the pyridine ring)
7.70 (1H, t, J = 8Hz, 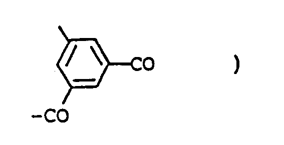
7.54 (1H, d, J = 6Hz, C₆-H)
7.31-7.07 (3H, m, 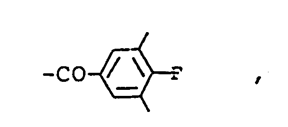
C₅-H of the pyridine ring)
5.97-5.89 (1H, m, C_{1'}-H)
4.34-3.84 (2H, m, C_{4'}-H)
2.47-1.86 (4H, m, C_{2'}-H, C_{3'}-H)

### Example 13

### Preparation of 3-[3-(6-benzoyloxy-3-cyano-2-pyridyloxycarbonyl)benzoyl]-5-fluoro-1-(2-tetrahydrofuranyl)uracil

The title compound was prepared in the same manner as in Example 5.
¹H-NMR(CDCl₃)δ:
8.69-8.12 (6H, m, 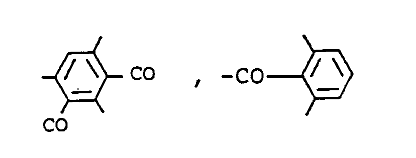
and C₄-H of the pyridine ring)
7.79-7.38 (6H, m, 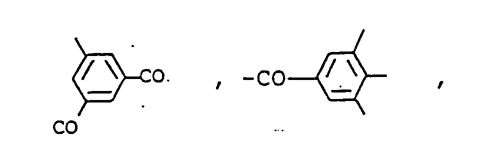
C₆-H, C₅-H of the pyridine ring)
5.96-5.87 (1H, m, C_{1'}-H)
4.30-3.80 (2H, m, C_{4'}-H)
2.50-1.85 (4H, m, C_{2'}-H, C_{3'}-H)

### Example 14

### Preparation of 3-[3-[3-cyano-6-(3,4,5-trimethoxybenzoyloxy)-2-pyridyloxycarbonyl]benzoyl]-5-fluoro-1-(2-tetrahydrofuranyl)uracil

The title compound was prepared in the same manner as in Example 5.
¹H-NMR(CDCl₃)δ:
8.69-8.21 (4H, m, 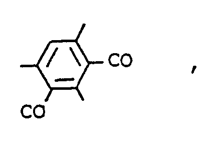
C₄-H of the pyridine ring)
7.73 (1H, t, J = 8Hz, 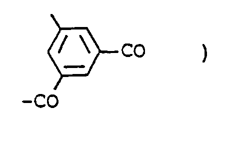
7.54 (1H, d, J = 6Hz, C₆-H)
7.44 (2H, s, 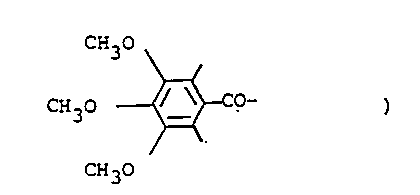
7.42 (1H, d, J = 8Hz, C₄-H of the pyridine ring)
5.97-5.90 (1H, m, C_{1'}-H)
4.34-3.81 (11H, m, C_{4'}-H and 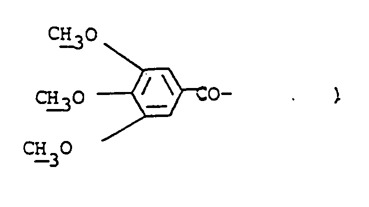
2.50-1.87 (4H, m, C_{2'}-H, C_{3'}-H)

### Example 15

### Preparation of 3-[3-[3-cyano-6-(2-furoyloxy)-2-pyridyloxycarbonyl]benzoyl]-5-fluoro-1-(2-tetrahydrofuranyl)uracil

The title compound was prepared in the same manner as in Example 5.
¹H-NMR(CDCl₃)δ:
8.68-8.20 (4H, m, 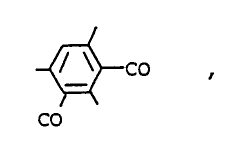
C₄-H of the pyridine ring)
7.81-7.40 (5H, m, 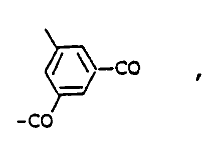
C₆-H, C₅-H of the pyridine ring and 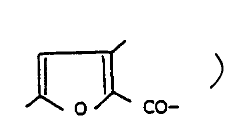
6.61 (1H, dd, J = 2Hz, J = 4Hz, 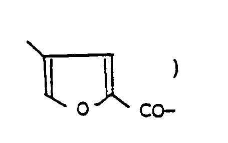
5.98-5.88 (1H, m, C_{1'}-H)
4.27-3.84 (2H, m, C_{4'}-H)
2.36-1.86 (4H, m, C_{2'}-H, C_{3'}-H)

### Example 16

### Preparation of 3-[4-(6-benzoyloxy-3-cyano-2-pyridyloxycarbonyl)-3-furoyl]-5-fluoro-1-(2-tetrahydrofuranyl)uracil

The title compound was prepared in the same manner as in Example 5.
¹H-NMR(CDCl₃)δ:
8.39 (2H, s, 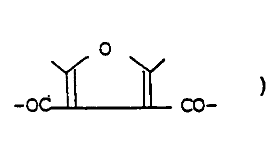
8.25-8.11 (3H, m, 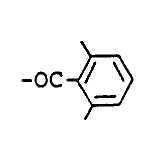
and C₄-H of the pyridine ring)
7.69-7.22 (5H, m, 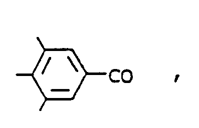
C₆-H and C₅-H of the pyridine ring)
5.92-5.82 (1H, m, C_{1'}-H)
4.22-3.71 (2H, m, C_{4'}-H)
2.33-1.68 (4H, m, C_{2'}-H, C_{3'}-H)

### Example 17

### Preparation of 3-[3-[3-cyano-6-(2-thenoyloxy)-2-pyridyloxycarbonyl]benzoyl]-5-fluoro-1-(2-tetrahydrofuranyl)uracil

The title compound was prepared in the same manner as in Example 5.
¹H-NMR(CDCl₃)δ:
8.66-8.18 (4H, m, 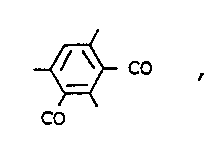
C₄-H of the pyridine ring)
8.03 (1H, dd, J = 1Hz, J = 4Hz, 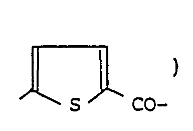
7.81-7.64 (2H, m, 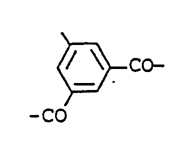
and 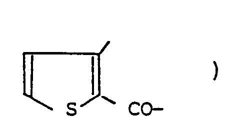
7.53 (1H, d, J = 6Hz, C₆-H)
7.44 (1H, d, J = 8Hz, C₅-H of the pyridine ring)
7.26-7.14 (1H, m, 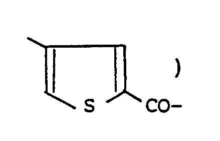
6.00-5.90 (1H, m, C_{1'}-H)
4.28-3.87 (2H, m, C_{4'}-H)
2.50-1.89 (4H, m, C_{2'}-H, C_{3'}-H)

### Example 18

### Preparation of 3-[3-[3-cyano-6-(2,4-dichlorobenzoyloxy)-2-pyridyloxycarbonyl]benzoyl]-5-fluoro-1-(2-tetrahydrofuranyl)uracil

The title compound was prepared in the same manner as in Example 5.
¹H-NMR(CDCl₃)δ:
8.67-8.21 (4H, m, 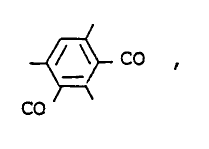
C₄-H of the pyridine ring)
8.09 (1H, d, J = 9Hz, 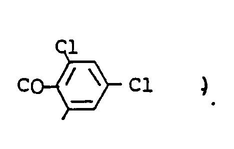
7.73 (1H, t, J = 8Hz, 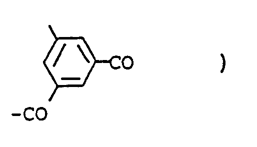
7.57-7.33 (4H, m, C₆-H, C₅-H of the pyridine ring and 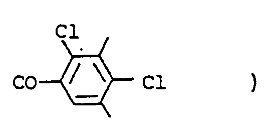
6.01-5.90 (1H, m, C_{1'}-H)
4.35-3.82 (2H, m, C_{4'}-H)
2.50-1.80 (4H, m, C_{2'}-H, C_{3'}-H)

### Example 19

### Preparation of 3-[5-(6-benzoyloxy-3-cyano-2-pyridyloxycarbonyl)-3-nicotincyl]-5-fluoro-1-(2-tetrahydrofuranyl)uracil

The title compound was prepared in the same manner as in Example 5.
¹H-NMR(CDCl₃)δ:
9.57-8.84 (3H, m, 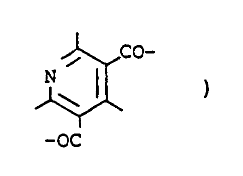
8.27 (1H, d, J = 8Hz, 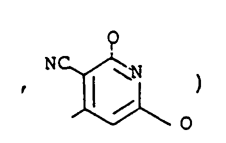
8.25-8.12 (2H, m, 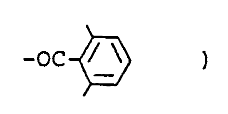
7.70-7.42 (4H, m, C₆-H and 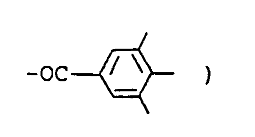
7.40 (1H, d, J = 8Hz, 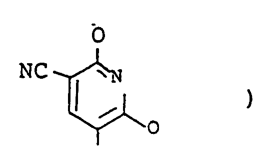
5.94-5.88 (1H, m, C_{1'}-H)
4.34-3.71 (2H, m, C_{4'}-H)
2.50-1.88 (4H, m, C_{2'}-H, C_{3'}-H)

### Example 20

### Preparation of 3-[3-(1,2-dihydro-2-oxo-6-pyridyloxycarbonyl)benzoyl]-5-fluoro-1-(2-tetrahydrofuranyl)uracil

Isophthaloyl chloride (1.50 g) and 6.0 ml of triethylamine were added to a solution of 1.00 g of 5-fluoro-1-(2-tetrahydrofuranyl)uracil in 50 ml of dioxane. The mixture was refluxed for 1.5 hours. The resulting precipitate was removed by filtration and the filtrate was concentrated. To the concentrate was added 50 ml of methylene chloride and thereto added was 10 ml of a solution of 1.50 g of 2,6-bis(trimethylsilyloxy)pyridine in methylene chloride. The mixture was left to stand at room temperature for 3 hours. The solvent was distilled off and the residue was subjected to silica gel column chromatography using as an eluent 2 % methanol-chloroform, giving 0.30 g of the title compound in a yield of 14 %.
¹H-NMR(CDCl₃)δ:
8.66-8.19 (3H, m, 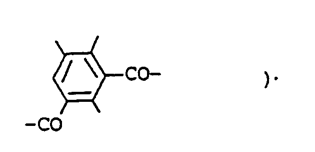
7.83-7.62 (2H, m, 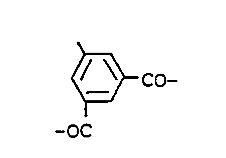
and C₄-H of the pyridine ring)
7.52 (1H, d, J = 6Hz, C₆-H)
6.77 and 6.68 (each 1H, d, J = 8Hz, C₃·₅-H of the pyridine ring)
5.99-5.92 (1H, m, C_{1'}-H)
4.30-3.88 (2H, m, C_{4'}-H)
2.44-1.89 (4H, m, C_{2'}·_{3'}-H) Pharmacological Test I
(a) Sarcoma-180 subcultured in an ascites of ICR mice was diluted with a physiological saline solution and subcutaneously transplanted into the backs of ICR mice in an amount of 2 X 10⁷ cells each. Twenty-four hours after the transplantation, a test compound suspended in a 5 % solution of gum arabic was orally administered to each of mice once a day for 7 consecutive days.

The solid tumor was extirpated from under the dorsal skin of mice on the 10th day after the transplantation to measure the weight of the tumor. There was determined the ratio (T/C) of the weight or tumor (T) cut out from the group of mice treated with the test compound to the weight of tumor (C) from the group of mice not treated therewith. The 50 % tumor inhibition dose (ED₅₀ value) in which T/C is 0.5 was determined from the dose-response curve of dosage and the ratio (T/C). Table 1 shows the results.

Table 1 below lists the results together with the value obtained by using as a control an anti-cancer preparation containing 5-fluoro-1-(2-tetrahydrofuranyl)uracil and uracil in a ratio by weight of 1 to 4.

**Table 1**

| Test Compound | ED₅₀ (mg/kg) |
|---|---|
| Compound of Example 1 | 10 |
| Compound of Example 3 | 25 |
| Compound of Example 4 | 18 |
| Compound of Example 10 | 28 |
| Compound of Example 15 | 18 |
| Control | 30 |

### Preparation Example 1

| | |
|---|---|
| Compound of Example 2 | 0.25 mg |
| Starch | 130 mg |
| Magnesium stearate | 20 mg |
| Lactose | 50 mg |
| Total | About 200 mg |

Tablets were prepared in a conventional manner which each had the foregoing composition.

### Preparation Example 2

| | |
|---|---|
| Compound of Example 1 | 12.5 mg |
| Polyethylene glycol (molecular weight: 4000) | 0.3 g |
| Sodium chloride | 0.9 g |
| Polyoxyethylene sorbitan monooleate | 0.4 g |
| Sodium methabisulfite | 0.1 g |
| Methylparaben | 0.18 g |
| Propylparaben | 0.02 g |
| Distilled water for injection | 100 ml |

In the distilled water were dissolved with stirring at 80°C the parabens, sodium methabisulfite, and sodium chloride. The resulting solution was cooled to 40°C. In the solution were dissolved the compound of this invention, polyethylene glycol and polyoxyethylene sorbitan monooleate in this order. To the solution was added the distilled water to adjust the amount to the desired level. Then the mixture was passed through filter paper for sterilization and placed into ampoules in an amount of 1 ml per ampoule to obtain an injection preparation.

## Claims

1. A compound represented by the formula 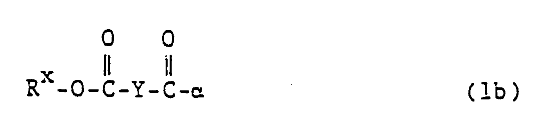 wherein R^{x} is a pyridyl group optionally having 1 to 4 substituents selected from the group consisting of hydroxy group, oxo group, halogen atom, amino group, carboxyl group, cyano group, nitro group, carbamoyl group, lower alkylcarbamoyl group, carboxy-lower alkylcarbamoyl group, phenyl-carbamoyl optionally substituted with 1 to 3 substituents selected from the group consisting of halogen atom, lower alkoxy group and lower alkyl group on the phenyl ring, lower alkoxycarbonyl-lower alkylcarbamoyl group, lower alkyl group, lower alkenyl group, lower alkoxycarbonyl group, tetrahydrofuranyl group, tetrahydropyranyl group, lower alkoxy-lower alkyl group, lower alkylthio-lower alkyl group, phenyl-lower alkoxy-lower alkyl group, phthalidyl group and acyloxy group, Y is an arylene group, and α is a known 5-fluorouracil derivative residue which is linked by an ester or amide linkage to the carbonyl group to which this substitutent α is attached and which is selected from
(i) a group formed from a 5-fluorouracil derivative represented by the formula 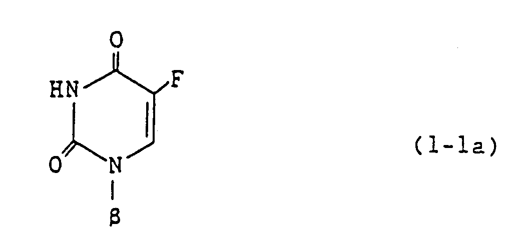 wherein β represents hydrogen atom, tetrahydrofuranyl, lower alkylcarbamoyl, phthalidyl, lower alkoxy-lower alkyl or lower alkanoyloxy-lower alkoxycarbonyl group, and
(ii) a group formed from a 5-fluorouracil derivative represented by the formula 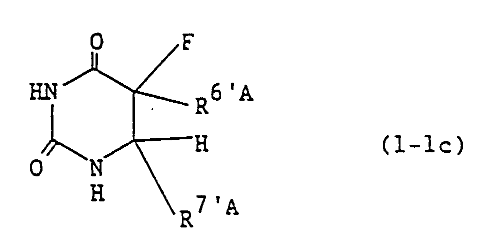 wherein R^{6'A} represents lower alkoxycarbonyl group, R^{7'A} represents lower alkoxy group or group

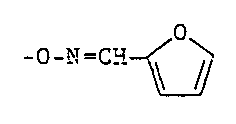

2. A compound according to claim 1 wherein the acyl of acyloxy group as the substituent of the pyridyl group represented by R^{x} is selected from the group consisting of:
(i) C₁-C₂₀ alkanoyl groups optionally substituted with the substituent selected from the group consisting of halogen atom, hydroxy group, lower alkoxy group, aryloxy group, substituted or unsubstituted aryl group, phenyl-lower alkoxycarbonyl group and lower alkylcarbamoyl group,
(ii) arylcarbonyl groups optionally substituted with lower alkylenedioxy group or with 1 to 3 substituents selected from the group consisting of halogen atom, lower alkyl group, lower alkoxy group, carboxy group, lower alkoxycarbonyl group, nitro group, cyano group, phenyl-lower alkoxycarbonyl group, hydroxy group, guanidyl group, phenyl-lower alkoxy group, amino group and amino group substituted with lower alkyl group,
(iii) 5- or 6- membered unsaturated hetero ring-carbonyl groups having nitrogen atom, sulfur atom or oxygen atom as the hetero atom,
(iv) carbonic acid ester residue such as aryloxycarbonyl groups, straight or branched-chain or cyclic alkoxycarbonyl groups,
(v) substituted or unsubstituted cycloalkylcarbonyl groups,
(vi) lower alkenyl (or lower alkynyl)carbonyl groups, and
(vii) lower alkenyl (or lower alkynyl)oxycarbonyl groups.

3. A compound according to claim 2 wherein the group 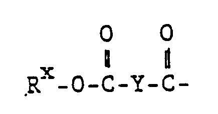 is a group represented by the formula 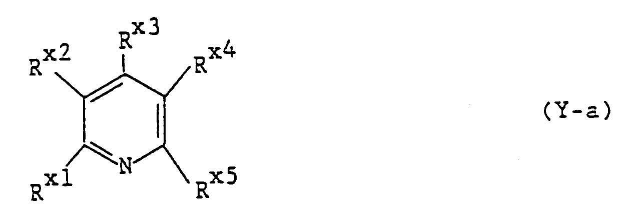 wherein R^{x1} is hydroxy or acyloxy; R^{x2} and R^{x4} are each hydrogen, halogen, amino, carboxyl, carbamoyl, cyano, nitro, lower alkyl, lower alkenyl or lower alkoxycarbonyl; R^{x3} and R^{x5} are each hydrogen, hydroxy or acyloxy; when at least one of R^{x1}, R^{x3} and R^{x5} is free hydroxy, the structure of 1-position on the pyridine ring can be 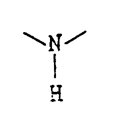 due to the keto-enol tautomerism, said hydrogen attached to the nitrogen being optionally substituted with a substituent selected from the group consisting of lower alkyl, tetrahydrofuranyl, tetrahydropyranyl, lower alkoxy-lower alkyl, phthalidyl, carbamoyl, lower alkoxycarbonyl-lower alkylcarbamoyl, phenyl-lower alkoxy-lower alkyl, phenylcarbamoyl which may have 1 to 3 substituents selected from the group consisting of halogen, lower alkoxy and lower alkyl on the phenyl ring, lower alkylcarbamoyl, carboxy-lower alkylcarbamoyl, lower alkylthio-lower alkyl and lower alkenyl, provided that at least one of R^{x1}, R^{x3} and R^{x5} represents hydroxy group and that the hydrogen of one hydroxyl group represented by R^{x1}, R^{x3} or R^{x5} is substituted with a group 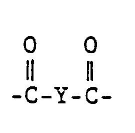 wherein Y is as defined above.

4. A compound according to claim 3 wherein α is a group formed from a 5-fluorouracil derivative represented by the formula 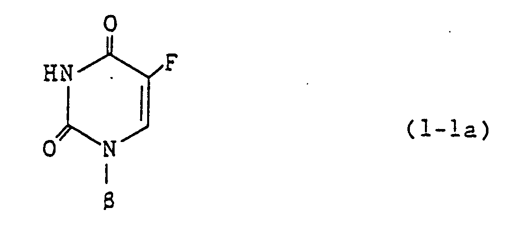 wherein β represents hydrogen atom, tetrahydrofuranyl, lower alkylcarbamoyl, phthalidyl, lower alkoxy-lower alkyl or lower alkanoyloxy-lower alkoxycarbonyl group.

5. A compound according to claim 3 wherein α is a group formed from a 5-fluorouracil derivative represented by the formula 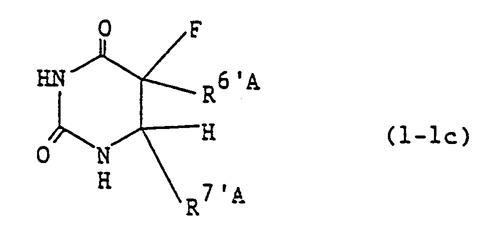 wherein R^{6'A} represents lower alkoxycarbonyl group, R^{7'A} represents lower alkoxy group or group

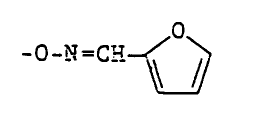

6. A compound according to any of claims 4 and 5 wherein the group 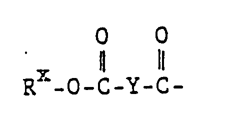 is a group represented by the formula 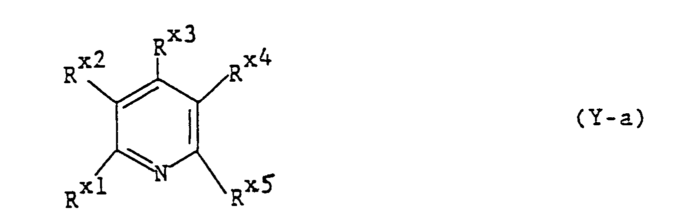 wherein R^{x1} is hydroxy or acyloxy; R^{x2} and R^{x4} are each hydrogen, halogen, amino, carboxyl, carbamoyl, cyano, nitro, lower alkyl, lower alkenyl or lower alkoxycarbonyl; R^{x3} and R^{x5} are each hydrogen, hydroxy or acyloxy, provided that at least one of R^{x1}, R^{x3} and R^{x5} represents hydroxy group and that the hydrogen of one hydroxy group represented by R^{x1}, R^{x3} or R^{x5} is substituted with a group 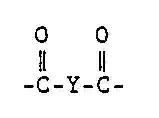 wherein Y is as defined above.

7. A compound according to claim 6 wherein R^{x1} is hydroxy, benzoyloxy, C₁-C₆ alkanoyloxy or furoyloxy, R^{x2} is hydrogen atom, one of R^{x3} and R^{x5} is hydrogen atom and the other or R^{x3} and R^{x5} is hydroxy, benzoyloxy, C₁-C₆ alkanoyloxy or furoyloxy, and R^{x4} is cyano group or halogen atom, provided that at least one of R^{x1}, R^{x3} and R^{x5} is hydroxy and that the hydrogen of one hydroxy group represented by R^{x1}, R^{x3} or R^{x5} is substituted with a group 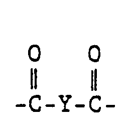 wherein Y is as defined above.

8. A compound according to claim 1 which is selected from the group consisting of 3-[3-(6-benzoyloxy-3-cyano-2-pyridyloxycarbonyl)benzoyl]-1-(2-tetrahydrofuranyl)-5-fluorouracil, 3-[3-(4-benzoyloxy-5-chloro-2-pyridyloxycarbonyl)benzoyl]-1-(2-tetrahydrofuranyl)-5-fluorouracil, 3-[3-(6-furoyloxy-3-cyano-2-pyridyloxycarbonyl)benzoyl]-1-(2-tetrahydrofuranyl)-5-fluorouracil, 3-[4-(6-furoyloxy-3-cyano-2-pyridyloxycarbonyl)-3-furoyl]-1-(2-tetrahydrofuranyl)-5-fluorouracil, 3-[4-(6-acetyloxy-3-cyano-2-pyridyloxycarbonyl)-3-furoyl]-1-(2-tetrahydrofuranyl)-5-fluorouracil and 3-{3-[5-chloro-1,2-dihydro-2-oxo-1-(2-tetrahydrofuranyl)-4-pyridyloxycarbonyl] benzoyl}-1-(2-tetrahydrofuranyl)-5-fluorouracil.

9. A process for preparing a compound represented by the formula 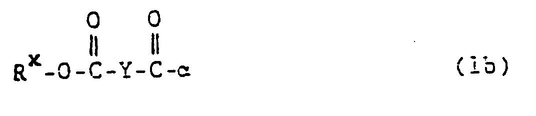 wherein R^{x}, Y and α are as defined in claim 1, characterized in that
a) the process comprises reacting a compound of the formula 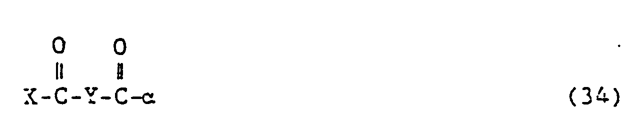 wherein X is halogen atom and Y and α are as defined above with a compound of the formula
R^{x}-O-R⁸ (26)
wherein R^{x} is as defined above, and R⁸ is hydrogen or tri(lower alkyl)silyl group, or
b) the process comprises reacting a compound of the formula 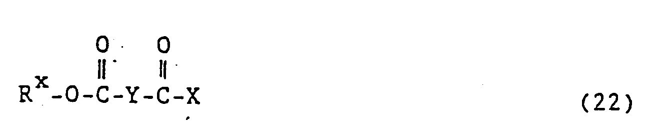 wherein R^{x} and Y are as defined above and X is halogen atom with a compound of the formula
αH (36)
wherein α is as defined above.

10. A compound having the formula (1b) according to claim 1 for use in the treatment of cancer.

11. A pharmazeutical composition comprising a compound having the formula (1b) according to claim 1 and a pharmaceutically acceptable carrier.

## Patentansprüche

1. Verbindung der Formel: 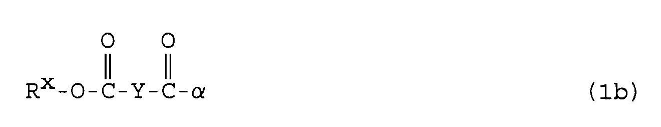 in der R^{x} eine Pyridylgruppe ist mit gegebenenfalls 1 bis 4 Substituenten, die aus der Gruppe ausgewählt sind, die besteht aus: einer Hydroxygruppe, Oxogruppe, einem Halogenatom, einer Aminogruppe, Carboxylgruppe, Cyanogruppe, Nitrogruppe, Carbamoylgruppe, einem Niederalkylcarbamoylrest, Carboxyniederalkylcarbamoylrest, einer Phenylcarbamoylgruppe, die an dem Phenylring gegebenenfalls 1 bis 3 Substituenten aufweist, die aus der Gruppe ausgewählt sind, die aus einem Halogenatom, einem Niederalkoxyrest und Niederalkylrest besteht, einem Niederalkoxycarbonyl-niederalkylcarbamoylrest, Niederalkylrest, Niederalkenylrest, Niederalkoxycarbonylrest, einer Tetrahydrofuranylgruppe, Tetrahydropyranylgruppe, einem Niederalkoxy-niederalkylrest, Niederalkylthio-niederalkylrest, Phenyl-niederalkoxy-niederalkylrest, einer Phthalidylgruppe und einem Acyloxyrest, Y ein Arylenrest ist und α ein bekannter 5-Fluoruracilderivatrest ist, der an die Carbonylgruppe durch eine Ester- oder Amidbindung, mit der dieser Substituent α verknüpft ist, gebunden ist, und der ausgewählt ist aus:
(i) einem aus einem 5-Fluoruracilderivat gebildeten Rest der Formel 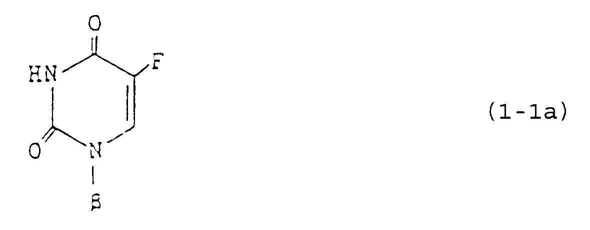 in der β ein Wasserstoffatom, eine Tetrahydrofuranylgruppe, ein Niederalkylcarbamoyl-, Phthalidyl-, Niederalkoxy-niederalkyl oder Niederalkanoyloxy-niederalkoxycarbonylrest ist und
(ii) einem aus einem 5-Fluoruracilderivat gebildeten Rest der Formel 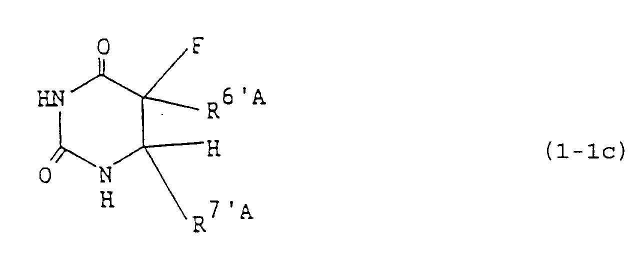 in der R^{6'A} einen Niederalkoxycarbonylrest darstellt und R^{7'A} einen Niederalkoxyrest oder eine 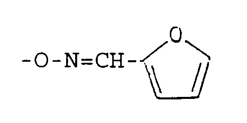 -Rest darstellt.

2. Verbindung gemäß Anspruch 1, in der der Acylrest des Acyloxyrests wie der durch R^{x} dargestellte Substituent der Pyridylgruppe aus der Gruppe ausgewählt wird, die besteht aus:
(i) (C₁₋₂₀)Alkanoylresten, die gegebenenfalls mit einem Substituenten substituiert sind, der ausgewählt ist aus der Gruppe, die aus einem Halogenatom, einer Hydroxygruppe, einem Niederalkoxyrest, Aryloxyrest, substituierten oder unsubstituierten Arylrest, Phenyl-niederalkoxycarbonylrest und Niederalkylcarbamoylrest besteht,
(ii) Arylcarbonylresten, die gegebenenfalls mit einem Niederalkylendioxyrest oder mit 1 bis 3 Substituenten substituiert sind, die ausgewählt sind aus der Gruppe, die aus einem Halogenatom, einem Niederalkylrest, Niederalkoxyrest, einer Carboxygruppe, einem Niederalkoxycarbonylrest, einer Nitrogruppe, Cyanogruppe, einem Phenyl-niederalkoxy-carbonylrest, einer Hydroxygruppe, Guanidylgruppe, einem Phenylniederalkoxyrest, einer Aminogruppe und einer mit einem Niederalkylrest substituierten Aminogruppe besteht,
(iii) 5- oder 6-gliedrigen ungesättigten Heteroring-carbonylresten mit einem Stickstoffatom, Schwefelatom oder Sauerstoffatom als Heteroatom,
(iv) Carbonsäureesterresten, wie Aryloxycarbonylresten, gerad- oder verzweigtkettigen oder cyclischen Alkoxycarbonylresten,
(v) substituierten oder unsubstituierten Cycloalkylcarbonylresten,
(vi) Niederalkenyl-(oder Niederalkinyl-)carbonylresten und
(vii) Niederalkenyl-(oder Niederalkinyl)oxycarbonylresten.

3. Verbindung gemäß Anspruch 2, in der der Rest 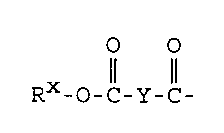 ein durch die Formel 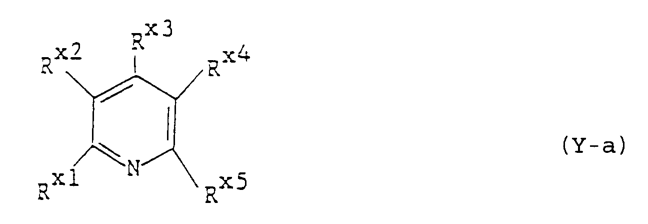 dargestellter Rest ist, in dem R^{x1} eine Hydroxygruppe oder ein Acyloxyrest ist; R^{x2} und R^{x4} jeweils ein Wasserstoffatom, Halogenatom, eine Amino-, Carboxyl-, Carbamoyl-, Cyano-, Nitrogruppe, ein Niederalkyl-, Niederalkenyl- oder Niederalkoxycarbonylrest sind; R^{x3} und R^{x5} jeweils ein Wasserstoffatom, eine Hydroxygruppe oder ein Acyloxyrest sind; und wenn mindestens einer der Reste R^{x1}, R^{x3} und R^{x5} eine freie Hydroxygruppe ist, kann die Struktur am Pyridinring in 1-Stellung infolge der Keto-Enol-Tautomerie 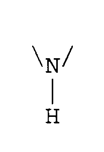 bedeuten,
wobei das mit dem Stickstoff verknüpfte Wasserstoffatom gegebenenfalls durch einen Substituenten substituiert sein kann, der ausgewählt ist aus der Gruppe, die besteht aus: einem Niederalkylrest, einer Tetrahydrofuranyl-, Tetrahydropyranylgruppe, einem Niederalkoxy-niederalkyl-, Phthalidyl-, Carbamoyl-, Niederalkoxycarbonyl-niederalkylcarbamoyl-, Phenylniederalkoxy-niederalkylrest, einem Phenylcarbamoylrest, der am Phenylring 1 bis 3 Substituenten aufweisen kann, ausgewählt aus der Gruppe, die aus einem Halogenatom, einem Niederalkoxyrest und einem Niederalkylrest besteht, einem Niederalkylcarbamoyl-, Carboxy-niederalkylcarbamoyl-, Niederalkylthio-niederalkyl- und Niederalkenylrest, mit der Maßgabe, daß mindestens einer der Reste R^{x1}, R^{x3} und R^{x5} eine Hydroxygruppe ist und daß das Wasserstoffatom einer durch R^{x1}, R^{x3} und R^{x5} dargestellten Hydroxylgruppen durch einen 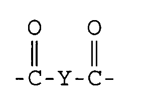 Rest substituiert ist, in dem Y wie vorstehend definiert ist.

4. Verbindung gemäß Anspruch 3, in der α einen aus einem 5-Fluoruracilderivat gebildeten Rest der Formel 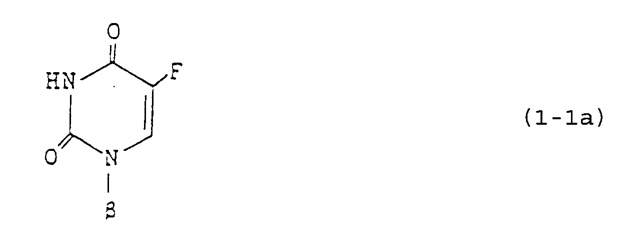 darstellt, in der β ein Wasserstoffatom, eine Tetrahydrofuranyl-, Niederalkylcarbamoyl-, Phthalidylgruppe, einen Niederalkoxyniederalkyl- oder Niederalkanoyloxy-niederalkoxycarbonylrest darstellt.

5. Verbindung gemäß Anspruch 3, in der α einen aus einem 5-Fluoruracilderivat gebildeten Rest der Formel 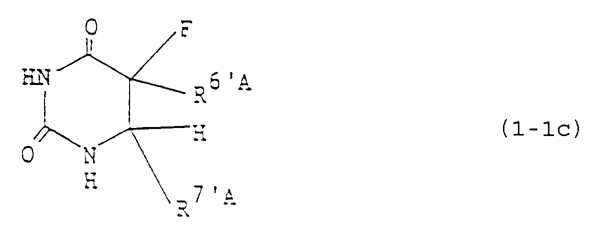 darstellt, in der R^{6'A} einen Niederalkoxycarbonylrest darstellt und R^{7'A} einen Niederalkoxyrest oder einen 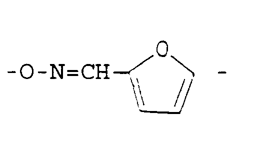 Rest darstellt.

6. Verbindung gemäß einem der Ansprüche 4 und 5, in der der Rest 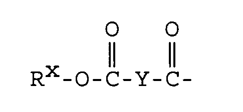 einen Rest der Formel 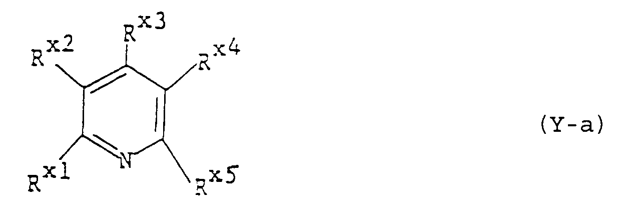 bedeutet, in der R^{x1} eine Hydroxygruppe oder ein Acyloxyrest ist; R^{x2} und R^{x4} jeweils ein Wasserstoff-, Halogenatom, eine Amino-, Carboxyl-, Carbamoyl-, Cyano-, Nitrogruppe, ein Niederalkyl-, Niederalkenyl- oder Niederalkoxycarbonylrest ist; R^{x3} und R^{x5} jeweils ein Wasserstoffatom, eine Hydroxygruppe oder ein Acyloxyrest sind, mit der Maßgabe, daß mindestens einer der Reste R^{x1}, R^{x3} und R^{x5} eine Hydroxygruppe ist und daß das Wasserstoffatom einer durch die Reste R^{x1}, R^{x3} und R^{x5} dargestellten Hydroxygruppen durch einen 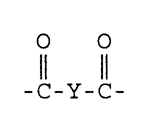 Rest substituiert ist, in dem Y wie vorstehend definiert ist.

7. Verbindung gemäß Anspruch 6, in der R^{x1} eine Hydroxy-, Benzoyloxygruppe, ein (C₁-C₆)Alkanoyloxy- oder Furoyloxyrest ist, R^{x2} ein Wasserstoffatom ist, einer der Reste R^{x3} und R^{x5} ein Wasserstoffatom und der andere der Reste R^{x3} und R^{x5} eine Hydroxy, Benzoyloxygruppe, ein (C₁-C₆)Alkanoyloxy- oder Furoyloxyrest ist und R^{x4} eine Cyanogruppe oder ein Halogenatom ist, mit der Maßgabe, daß mindestens einer der Reste R^{x1}, R^{x3} und R^{x5} eine Hydroxygruppe ist und daß das Wasserstoffatom einer durch die Reste R^{x1}, R^{x3} und R^{x5} dargestellten
Hydroxygruppe durch einen 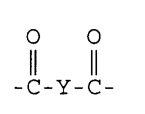 Rest substituiert ist, in dem Y wie vorstehend definiert ist.

8. Verbindung gemäß Anspruch 1, die aus der Gruppe ausgewählt ist, die aus 3-[3-(6-Benzoyloxy-3-cyano-2-pyridyloxycarbonyl)benzoyl]-1-(2-tetrahydrofuranyl)-5-fluoruracil, 3-[3-(4-Benzoyloxy-5-chlor-2-pyridyloxycarbonyl)benzoyl]-1-(2-tetrahydrofuranyl)-5-fluoruracil, 3-[3-(6-Furoyloxy-3-cyano-2-pyridyloxycarbonyl)benzoyl]-1-(2-tetrahydrofuranyl)-5-fluoruracil, 3-[4-(6-Furoyloxy-3-cyano-2-pyridyloxycarbonyl)-3-furoyl]-1-(2-tetrahydrofuranyl)-5-fluoruracil, 3-[4-(6-Acetyloxy-3-cyano-2-pyridyloxycarbonyl)-3-furoyl]-1-(2-tetrahydrofuranyl)-5-fluoruracil und 3-{3-[5-Chlor-1,2-dihydro-2-oxo-1-(2-tetrahydrofuranyl)-4-pyridyloxycarbonyl]benzoyl}-1-(2-tetrahydrofuranyl)-5-fluoruracil besteht.

9. Verfahren zur Herstellung einer Verbindung der Formel 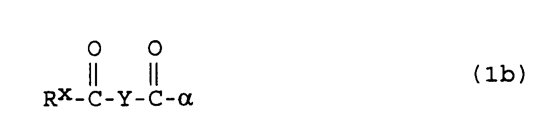 in der R^{x}, Y und α wie in Anspruch 1 definiert sind, gekennzeichnet dadurch, daß
a) das Verfahren die Umsetzung einer Verbindung der Formel 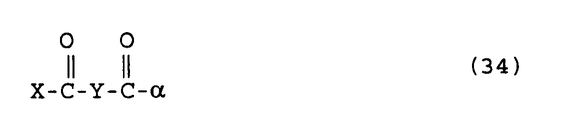 in der X ein Halogenatom ist und Y und α wie vorstehend definiert sind,
mit einer Verbindung der Formel
R^{x}-O-R⁸ (26)
in der R^{x} wie vorstehend definiert ist und R⁸ ein Wasserstoffatom oder ein Tri(nieder alkyl)silylrest ist, umfaßt, oder
b) das Verfahren die Umsetzung einer Verbindung der Formel 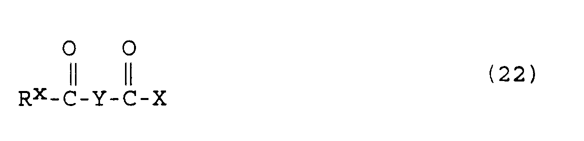 in der R^{x} und Y wie vorstehend definiert sind und X ein Halogenatom ist,
mit einer Verbindung der Formel
α-H (36)
in der α wie vorstehend definiert ist,
umfaßt.

10. Verbindung der Formel (1b) gemäß Anspruch 1 zur Verwendung bei der Krebsbehandlung.

11. Arzneimittel, das eine die Formel (1b) aufweisende Verbindung gemäß Anspruch 1 und einen pharmazeutisch verträglichen Träger umfaßt.

## Revendications

1. Composé répondant à la formule 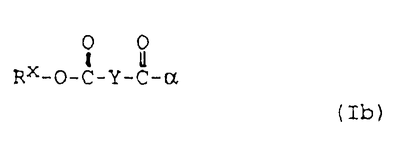 dans laquelle R^{x} représente un groupe pyridyle ayant optionnellement 1 à 4 substituants choisis dans le groupe consistant en un groupe hydroxy, un groupe oxo, un atome d'halogène, un groupe amino, un groupe carboxyle, un groupe cyano, un groupe nitro, un groupe carbamoyle, un groupe alkylcarbamoyle inférieur, un groupe carboxyalkylcarbamoyle inférieur, un phényle-carbamoyle optionnellement substitué par 1 à 3 substituants sélectionnés dans le groupe consistant en un atome d'halogène, un groupe alcoxy inférieur et un groupe alkyle inférieur sur le cycle phényle, un groupe alcoxycarbonyle inférieur-alkyle inférieur-carbamoyle, un groupe alkyle inférieur, un groupe alkényle inférieur, un groupe alcoxycarbonyle inférieur, un groupe tétrahydrofuranyle, un groupe tétrahydropyranyle, un groupe alcoxy inférieur-alkyle inférieur, un groupe alkylthio inférieur-alkyle inférieur, un groupe phénylealcoxy inférieur-alkyle inférieur, un groupe phtalidyle et un groupe acyloxy,
Y représente un groupe arylène et α est un groupe formé d'un dérivé de 5-fluorouracile connu qui est lié par une liaison ester ou amide au groupe carbonyle auquel ce substituant α est attaché et qui est sélectionné parmi
(i) un groupe formé à partir d'un dérivé de 5-fluorouracile répondant à la formule 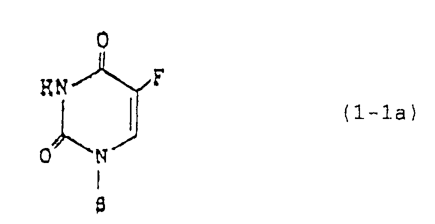 dans laquelle β représente un atome d'hydrogène, un groupe tétrahydrofuranyle, un alkylcarbamoyle inférieur, un phtalidyle, un alcoxy inférieur-alkyle inférieur ou un alcanoyloxy inférieur-alcoxycarbonyle inférieur, et
(ii) un groupe formé d'un dérivé de 5-fluorouracile répondant à la formule 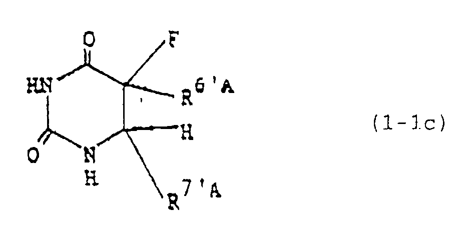 dans laquelle R^{6'A} représente un groupe alcoxycarbonyle inférieur, R^{7'A} représente un groupe alcoxy inférieur ou un groupe

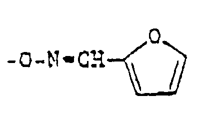

2. Composé selon la Revendication 1, caractérisé en ce que l'acyle du groupe acyloxy en tant que substituant du groupe pyridyle représenté par R^{x} est sélectionné dans le groupe consistant en :
(i) des groupes alcanoyles en C₁-C₂₀ optionnellement substitués avec un substituant sélectionné dans le groupe consistant en un atome d'halogène, un groupe hydroxy, un groupe alcoxy inférieur, un groupe aryloxy, un groupe aryle substitué ou non, un groupe phényle-alcoxycarbonyle inférieur et un groupe alkylcarbamoyle inférieur,
(ii) des groupes arylcarbonyles optionnellement substitués par un groupe alkylènedioxy inférieur ou par 1 à 3 substituants sélectionnés dans le groupe consistant en un atome d'halogène, un groupe alkyle inférieur, un groupe alcoxy inférieur, un groupe carboxy, un groupe alcoxycarbonyle inférieur, un groupe nitro, un groupe cyano, un groupe phényle-alcoxycarbonyle inférieur, un groupe hydroxy, un groupe guanidyle, un groupe phényle-alcoxy inférieur, un groupe amino et un groupe amino substitué par un groupe alkyle inférieur,
(iii) des groupes hétérocycliques insaturés à 5 ou 6 chaînons-carbonyle ayant un atome d'azote, un atome de soufre ou un atome d'oxygène comme hétéroatome,
(iv) un résidu ester d'acide carbonique tel que les groupes aryloxycarbonyles, à chaîne droite ou ramifiée ou des groupes alcoxycarbonyles cycliques,
(v) des groupes cycloalkylcarbonyles substitués ou non,
(vi) des groupes alkényles inférieurs (ou alkynyles inférieurs) carbonyles, et
(vii) des groupes alkényles inférieurs (ou alkynyles inférieurs) oxycarbonyles.

3. Un composé selon la Revendication 2, caractérisé en ce que le groupe 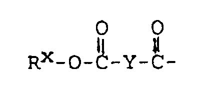 est un groupe répondant à la formule : 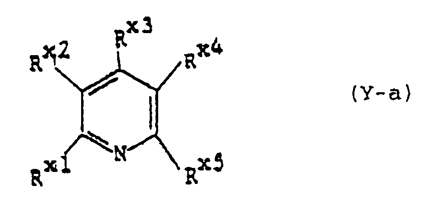 dans laquelle R^{x1} est un hydroxy ou un acyloxy ; R^{x2} et R^{x4} sont chacun un hydrogène, un halogène, un amino, un carboxyle, un carbamoyle, un cyano, un nitro, un alkyle inférieur, un alkényle inférieur ou un alcoxycarbonyle inférieur ;
R^{x3} et R^{x5} sont chacun un hydrogène, un hydroxy ou un acyloxy ; lorsque au moins l'un des groupes R^{x1}, R^{x3} et R^{x5} est un hydroxy libre, la structure en position 1 sur le cycle pyridine peut être 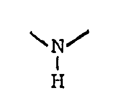 en raison du tautomérisme céto-énol, l'hydrogène attaché à l'azote étant optionnellement substitué par un substituant sélectionné dans le groupe consistant en un groupe alkyle inférieur, un groupe tétrahydrofuranyle, un groupe tétrahydropyranyle, un groupe alcoxy inférieur-alkyle inférieur, un groupe phtalidyle, un groupe carbamoyle, un groupe alcoxycarbonyle inférieur-alkylcarbamoyle inférieur, un groupe phényle-alcoxy inférieur-alkyle inférieur, un groupe phényl-carbamoyle qui peut avoir 1 à 3 substituants sélectionnés dans le groupe consistant en halogène, alcoxy inférieur, et alkyle inférieur sur le cycle phényle, alkylcarbamoyle inférieur, carboxyalkylcarbamoyle inférieur, alkylthio inférieur-alkyle inférieur et alkényle inférieur, à condition qu'au moins l'un des R^{x1}, R^{x3} et R^{x5} représente un groupe hydroxy et que l'hydrogène d'un groupe hydroxyle représenté par R^{x1}, R^{x3} ou R^{x5} est substitué par un groupe 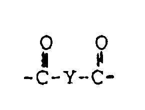 dans lequel Y est tel que défini ci-dessus.

4. Composé selon la Revendication 3, caractérisé en ce que α est un groupe formé d'un dérivé de 5-fluorouracile répondant à la formule 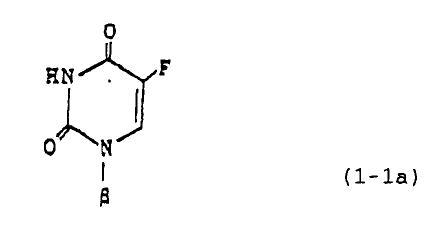 dans laquelle β représente un atome d'hydrogène, un groupe tétrahydrofuranyle, alkylcarbamoyle inférieur, phtalidyle, alcoxy inférieur-alkyle inférieur ou alcanoyloxy inférieur-alcoxycarbonyle inférieur.

5. Un composé selon la Revendication 3, caractérisé en ce que α est un groupe formé d'un dérivé de 5-fluorouracile répondant à la formule (1-1c) : 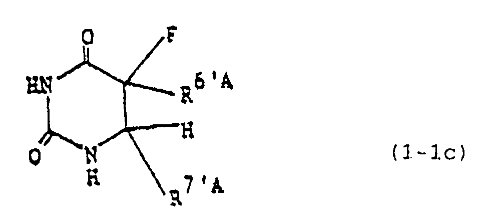 dans laquelle R^{6'A} représente un groupe alcoxycarbonyle inférieur, R^{7'A} représente un groupe alcoxy inférieur ou un groupe

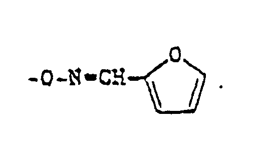

6. Un composé selon l'une quelconques des Revendications 4 et 5, caractérisé en ce que le groupe 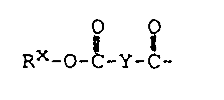 est un groupe répondant à la formule : 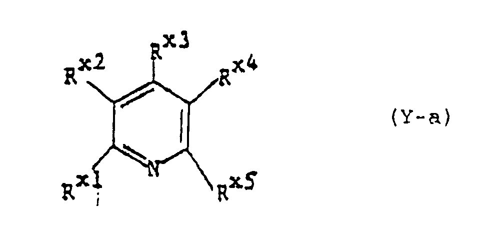 dans laquelle R^{x1} est hydroxy ou un acyloxy ;
R^{x2} et R^{x4} sont chacun un hydrogène, un halogène, un amino, un carboxyle, un carbamoyle, un cyano, un nitro, un alkyle inférieur, un alkényle inférieur ou un alcoxycarbonyle inférieur ;
R^{x3} et R^{x5} sont chacun un hydrogène, un hydroxy ou un acyloxy ; à condition qu'au moins l'un des groupes R^{x1}, R^{x3} et R^{x5} représente un groupe hydroxy et que l'hydrogène d'un groupe hydroxy représenté par R^{x1},
R^{x3} ou R^{x5} est substitué par un groupe 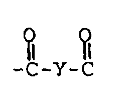 dans lequel Y est tel que défini ci-dessus.

7. Un composé selon la Revendication 6, caractérisé en ce que R^{x1} est un hydroxy, un benzoyloxy, un alcanoyloxy en C₁-C₆ ou un furoyloxy, R^{x2} est un atome d'hydrogène, l'un des R^{x3} et R^{x5} est un atome d'hydrogène et l'autre des R^{x3} et R^{x5} est un hydroxy, un benzyloxy, un alcanoyloxy en C₁-C₆ ou un furoyloxy, et R^{x4} est un groupe cyano ou un atome d'halogène, à condition qu'au moins l'un des R^{x1}, R^{x3} et R^{x5} est un hydroxy et que l'hydrogène d'un groupe hydroxy représenté par R^{x1}, R^{x3} ou R^{x5} est substitué par un groupe 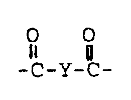 dans lequel Y est tel que défini ci-dessus.

8. Un composé selon la Revendication 1, caractérisé en ce qu'il est sélectionné dans le groupe consistant en 3-[3-(6-benzoyloxy-3-cyano-2-pyridyloxycarbonyl) benzoyl]-1-(2-tétrahydrofuranyl)-5-fluorouracile, 3-[3-(4-benzoyloxy-5-chloro-2-pyridyloxycarbonyl) benzoyl]-1-(2-tétrahydrofuranyl)-5-fluorouracile, 3-[3-(6-furoyloxy-3-cyano-2-pyridyloxycarbonyl) benzoyl]-1-(2-tétrahydrofuranyl)-5-fluorouracile, 3-[4-(6-furoyloxy-3-cyano-2-pyridyloxycarbonyl)-3-furoyl]-1-(2-tétrahydrofuranyl)-5-fluorouracile, 3-[4-(6-acétyloxy-3-cyano-2-pyridyloxycarbonyl)-3-furoyl]-1-(2-tétrahydrofuranyl)-5-fluorouracile et 3-{3-[5-chloro-1,2-dihydro-2-oxo-1-(2-tétrahydrofuranyl)-4-pyridyloxycarbonyl]benzoyl}-1-(2-tétrahydrofuranyl)-5-fluorouracile.

9. Procédé de préparation d'un composé répondant à la formule 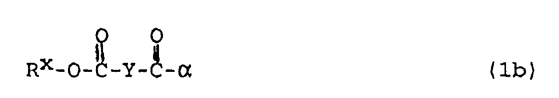 dans laquelle R^{x}, Y et α sont tels que définis dans la revendication 1, caractérisé en ce que :
a) le procédé comprend la réaction d'un composé de formule 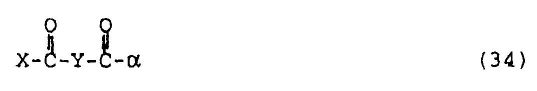 dans laquelle X est un atome d'halogène et Y et α sont tels que définis ci-dessus, avec un composé de formule
R^{x}-O-R⁸ (26)
dans laquelle R^{x} est tel que défini ci-dessus et R⁸ est un hydrogène ou un groupe tri(alkyle inférieur)silyle ou
b) le procédé comprend la réaction d'un composé de formule 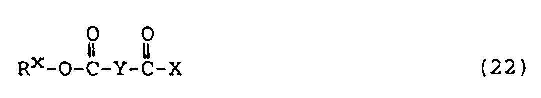 dans laquelle R^{x} et Y sont tels que définis ci-dessus et X est un atome d'halogène, avec un composé de formule
αH (36)
dans laquelle α est tel que défini ci-dessus.

10. Composé de formule (1b) selon la Revendication 1 pour son utilisation dans le traitement du cancer.

11. Composition pharmaceutique comprenant un composé de formule (1b) selon la Revendication 1 et un véhicule pharmaceutiquement acceptable.
